(19) Europäisches Patentamt / European Patent Office / Office européen des brevets

(11) EP 3 950 063 A1

(12) **EUROPEAN PATENT APPLICATION**
published in accordance with Art. 153(4) EPC

(43) Date of publication:
**09.02.2022 Bulletin 2022/06**

(21) Application number: **20784568.6**

(22) Date of filing: **30.03.2020**

(51) International Patent Classification (IPC):
***A61P 35/00*** (2006.01) ***A61P 35/02*** (2006.01)
***A61P 43/00*** (2006.01) ***C07D 471/04*** (2006.01)
***C07D 519/00*** (2006.01) ***A61K 31/437*** (2006.01)
***A61K 31/4427*** (2006.01) ***A61K 31/506*** (2006.01)
***A61K 31/5377*** (2006.01) ***A61K 31/5386*** (2006.01)

(52) Cooperative Patent Classification (CPC):
**A61K 31/437; A61K 31/4427; A61K 31/506; A61K 31/5377; A61K 31/5386; A61P 35/00; A61P 35/02; A61P 43/00; C07D 471/04; C07D 519/00**

(86) International application number:
**PCT/JP2020/014475**

(87) International publication number:
**WO 2020/203950 (08.10.2020 Gazette 2020/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.03.2019 JP 2019068233**

(71) Applicant: **KOWA COMPANY, LTD.**
**Naka-ku**
**Nagoya-shi**
**Aichi 460-8625 (JP)**

(72) Inventors:
- **WATANABE, Gen**
  **Higashimurayama-shi, Tokyo 189-0022 (JP)**
- **MORIMOTO, Toshiharu**
  **Higashimurayama-shi, Tokyo 189-0022 (JP)**
- **IWATA, Akira**
  **Higashimurayama-shi, Tokyo 189-0022 (JP)**
- **SASAKI, Hirotaka**
  **Higashimurayama-shi, Tokyo 189-0022 (JP)**
- **OGAMINO, Takahisa**
  **Higashimurayama-shi, Tokyo 189-0022 (JP)**
- **USUDA, Kosuke**
  **Higashimurayama-shi, Tokyo 189-0022 (JP)**
- **OKUDA, Ayumu**
  **Higashimurayama-shi, Tokyo 189-0022 (JP)**
- **ISHIWATA, Hiroyuki**
  **Higashimurayama-shi, Tokyo 189-0022 (JP)**
- **TABUNOKI, Yuichiro**
  **Higashimurayama-shi, Tokyo 189-0022 (JP)**
- **NISHII, Keigo**
  **Higashimurayama-shi, Tokyo 189-0022 (JP)**

(74) Representative: **Hartz, Nikolai**
**Wächtershäuser & Hartz**
**Patentanwaltspartnerschaft mbB**
**Weinstrasse 8**
**80333 München (DE)**

(54) **NOVEL AZAINDOLE DERIVATIVE**

(57) To provide a novel compound or a salt thereof or a solvate thereof, having a CSF-1R inhibitory activity and exhibiting antitumor effect.

An azaindole derivative of formula (I):

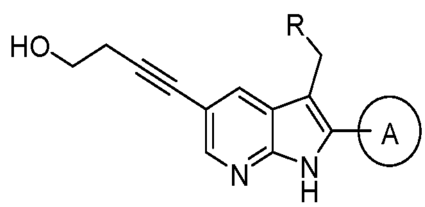

(I)

wherein

A represents a $C_{6-10}$ aryl ring, an aromatic heterocycle, or an unsaturated heterocycle, and
A optionally has one substituent or more same or different substituents; and
R represents a $C_{1-3}$ alkyl group or a saturated heterocyclic group,
or a salt thereof or a solvate thereof.

## Description

Technical Field

The present invention relates to a novel azaindole derivative and its pharmaceutical use.

Background Art

Colony-stimulating factor 1 (also referred to as macrophage-colony-stimulating factor (M-CSF)) regulates the activities of monocytes and macrophages and is involved in the survival, proliferation, and differentiation thereof (Non Patent Literatures 1 and 2). Since an increase in expression of the CSF-1 gene is observed in almost all tenosynovial giant cell tumor (TGCT), in recent years, a receptor thereof, i.e., colony-stimulating factor 1 receptor (CSF-1R), has become a target of antitumor agents (Non Patent Literatures 3 to 6) .

CSF-1R is a receptor-type tyrosine kinase belonging to the PDGF (platelet-derived growth factor) family and mediates the biological effects of CSF-1 (Patent Literature 1). Accordingly, a drug that inhibits the kinase activity of CSF-1R could be a new therapeutic agent for an immune disease or inflammatory disease caused by CSF-1.

For example, it has been reported that a high therapeutic effect was obtained by administering a CSF-1R inhibitor, PLX3397, to giant cell tumor of tendon sheath of which onset is triggered by overexpression of CSF-1 (Non Patent Literatures 3 and 7). In addition, since a correlation with acute leukemia has been reported, CSF-1R inhibitors are also expected as a therapeutic agent for acute leukemia (Non Patent Literatures 8 and 9, and Patent Literature 2).

As a kinase inhibitor having an azaindole skeleton, for example, a compound having a RET tyrosine kinase regulatory effect (Patent Literature 3) and a compound having a c-Kit tyrosine kinase inhibitory activity (Patent Literature 4) have been reported.

However, the compounds described in these Patent Literatures differ from the compounds of the present invention completely in substituents. In addition, these Patent Literatures do not describe the CSF-1R inhibitory effect, either.

Citation List

Patent Literatures

Patent Literature 1: WO 2011/107553
Patent Literature 2: WO 2009/075344
Patent Literature 3: WO 2005/062795
Patent Literature 4: WO 2006/009755

Non-Patent Literatures

Non-Patent Literature 1: Critical Reviews in Clinical Laboratory Sciences, 2012, 49, 49-61
Non-Patent Literature 2: Current Opinion in Immunology, 2006, 18, 39-48
Non-Patent Literature 3: New England Journal of Medicine, 2015, 373, 428-437
Non-Patent Literature 4: Cell, 2010, 141, 39-51
Non-Patent Literature 5: Frontiers in Immunology, 2014, 5, 489, 1-15
Non-Patent Literature 6: Current Opinion in Pharmacology, 2015, 23, 45-51
Non-Patent Literature 7: Proc. Natl. Acad. Sci. USA, 2006, 103, 690-695
Non-Patent Literature 8: Proc. Natl. Acad. Sci. USA, 1990, 87, 1377-1380
Non-Patent Literature 9: Nature Medicine, 2010, 16, 580-585

Summary of the Invention

Problem

The present invention aims to provide a novel azaindole derivative having a CSF-1R inhibitory effect.

In view of the above circumstances, the present inventors energetically conducted researches to find that the azaindole derivative of formula (I) below has a potent CSF-1R inhibitory effect and shows an antitumor effect. The

present inventors completed the present invention on the basis of the findings.

The present invention relates to the following [1] to [12].

[1] A compound of formula (I):

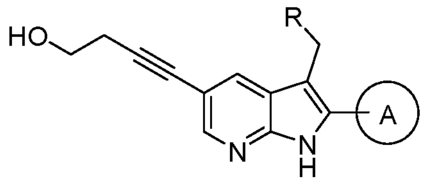

(I)

wherein
A represents a $C_{6-10}$ aryl ring, an aromatic heterocycle, or an unsaturated heterocycle, and A optionally has one substituent or two or more same or different substituents; and
R represents a $C_{1-3}$ alkyl group or a saturated heterocyclic group,

or a salt thereof or a solvate thereof.

[2] The compound or the salt thereof, or the solvate thereof according to [1], wherein A is a benzene ring, a naphthalene ring, a pyridine ring, a pyrimidine ring, a 1,2-dihydropyridine ring, a 1,2,3,4-tetrahydropyridine ring, or a 1,2,3,6-tetrahydropyridine ring; and the substituent of A is one to three selected from the group consisting of a $C_{1-3}$ alkyl group, a $C_{1-3}$ alkyloxy group, a $C_{1-3}$ alkylcarbonyl group, a hydroxypiperidinyl group, an oxetanyloxy group, and a morpholinyl group.

[3] The compound or the salt thereof, or the solvate thereof according to [1] or [2], wherein R is a $C_{1-3}$ alkyl group, a tetrahydropyran ring, a morpholine ring, or a 3-oxa-8-azabicyclo[3.2.1]octane ring.

[4] A pharmaceutical composition comprising the compound or the salt thereof, or the solvate according to any one of [1] to [3].

[5] A CSF-1R inhibitor comprising the compound or the salt thereof, or the solvate thereof according to any one of [1] to [3] as an active component.

[6] An anticancer agent comprising the compound or the salt thereof, or the solvate thereof according to any one of [1] to [3] as an active component.

[7] Use of the compound or the salt thereof, or the solvate thereof according to any one of [1] to [3] for manufacturing a CSF-1R inhibitor.

[8] Use of the compound or the salt thereof, or the solvate thereof according to any one of [1] to [3] for manufacturing an anticancer agent.

[9] The compound or the salt thereof, or the solvate thereof according to any one of [1] to [3] for inhibiting CSF-1R.

[10] The compound or the salt thereof, or the solvate thereof according to any one of [1] to [3] for preventing or treating cancer.

[11] A method for inhibiting CSF-1R, comprising administering the compound or the salt thereof, or the solvate thereof according to any one of [1] to [3] to a patient in need thereof.

[12] A method for treating cancer, comprising administering the compound or the salt thereof, or the solvate thereof according to any one of [1] to [3] to a patient in need thereof.

## Effect of the Invention

The present invention provides a novel azaindole derivative having an excellent CSF-1R inhibitory effect, and which is useful as an anticancer agent.

## Brief Description of Drawing

Figure 1 shows results of Test Example 2, i.e., the change in the tumor volume on each measurement day when an inventive compound was orally administered to a human non-small cell lung cancer cell line NCI-H460 subcutaneously transplanted nude mouse model. In the graph, the horizontal axis represents the days after cell transplantation, and the vertical axis represents the tumor volume in each group. In addition, ** indicates that there is a significant difference of $p < 0.01$ for the Control group, and *** indicates that there is a significant difference of $p < 0.001$ for the Control group (both are Student's t tests).

Description of Embodiments

In the present specification, the term "$C_{1-3}$ alkyl group" means a linear or branched alkyl group having 1 to 3 carbon atoms, and examples thereof include a methyl group, an ethyl group, an n-propyl group, and an isopropyl group.
In the present specification, the term "$C_{1-3}$ alkyloxy group" means a linear or branched alkyl group having 1 to 3 carbon atoms, and examples thereof include a methoxy group, an ethoxy group, an n-propyloxy group, and an isopropyloxy group.
In the present specification, the term "$C_{1-3}$ alkylcarbonyl group" means a carbonyl group to which a linear or branched alkyl group having 1 to 3 carbon atoms is bonded, and examples thereof include an acetyl group, a propanoyl group, an n-butanoyl group, and an isobutanoyl group.
In the present specification, the term "$C_{6-10}$ aryl ring" means a cyclic chemical structure that is composed of 6 to 10 carbon atoms and shows aromaticity, and examples thereof include a benzene ring and a naphthalene ring.
In the present specification, the term "aromatic heterocycle" means a cyclic chemical structure that is composed of two or more elements and shows aromaticity, and examples thereof include a pyrrole ring, an imidazole ring, a pyrazole ring, an isothiazole ring, an isoxazole ring, a pyridine ring, a pyrazine ring, a pyrimidine ring, a pyridazine ring, an indolizine ring, an indole ring, an isoindole ring, an indazole ring, a pyrrolopyridine ring, a purine ring, a quinoline ring, an isoquinoline ring, a phthalazine ring, a naphthyridine ring, a quinoxaline ring, a quinazoline ring, a cinnoline ring, and a pteridine ring.
In the present specification, the term "saturated heterocycle" means a cyclic chemical structure that is composed of two or more elements and does not include an unsaturated bond, and examples thereof include a pyrrolidine ring, an imidazolidine ring, a pyrazolidine ring, an oxetane ring, a piperidine ring, a piperazine ring, a tetrahydrofuran ring, a tetrahydropyran ring, a morpholine ring, and a 3-oxa-8-azabicyclo[3.2.1]octane ring.
In the present specification, the "unsaturated heterocycle" means a cyclic chemical structure that is composed two or more elements and includes one or more unsaturated bonds, and examples thereof include a 1,2-dihydropyridine ring, a 1,2,3,4-tetrahydropyridine ring, and a 1,2,3,6-tetrahydropyridine ring.
In formula (I), the "$C_{6-10}$ aryl ring" as A is preferably a benzene ring or a naphthalene ring and more preferably a benzene ring.
In formula (I), the "aromatic heterocycle" as A is preferably a pyridine ring or a pyrimidine ring and more preferably a pyridine ring.
In formula (I), the "unsaturated heterocycle" as A is preferably a 1,2-dihydropyridine ring, a 1,2,3,4-tetrahydropyridine ring, or a 1,2,3,6-tetrahydropyridine ring and more preferably a 1,2,3,6-tetrahydropyridine ring.
In formula (I), the "substituent" in A is preferably a $C_{1-3}$ alkyl group, a $C_{1-3}$ alkyloxy group, a $C_{1-3}$ alkylcarbonyl group, a hydroxypiperidinyl group, an oxetanyloxy group, or a morpholinyl group and more preferably a methyl group, a methoxy group, an acetyl group, a 4-hydroxypiperidinyl group, an oxetan-3-yloxy group, or a morpholino group, but is not particularly limited thereto. In addition, the number of substituents in A is preferably 1 to 3, where the substituents may be the same or different.
In formula (I), the "$C_{1-3}$ alkyl group" as R is preferably an isopropyl group.
In formula (I), the "saturated heterocycle" as R is preferably a tetrahydropyran ring, a morpholine ring, or a 3-oxa-8-azabicyclo[3.2.1]octane ring.
Examples of the compound of formula (I) include the following compounds:
4-(2-(4-methoxyphenyl)-3-(morpholinomethyl)-1H-pyrrolo[2,3-b]pyridin-5-yl)but-3-yn-1-ol (Example 1);
4-(3-((3-oxa-8-azabicyclo[3.2.1]octan-8-yl)methyl)-2-(4-methoxyphenyl)-1H-pyrrolo[2,3-b]pyridin-5-yl)but-3-yn-1-ol (Example 2);
4-(2-(3,4-dimethoxyphenyl)-3-(morpholinomethyl)-1H-pyrrolo[2,3-b]pyridin-5-yl)but-3-yn-1-ol (Example 3);
4-(3-(morpholinomethyl)-2-(4-morpholinophenyl)-1H-pyrrolo[2,3-b]pyridin-5-yl)but-3-yn-1-ol (Example 4);
1-(4-(5-(4-hydroxybut-1-yn-1-yl)-3-(morpholinomethyl)-1H-pyrrolo[2,3-b]pyridin-2-yl)phenyl)piperidin-4-ol (Example 5);
4-(3-((3-oxa-8-azabicyclo[3.2.1]octan-8-yl)methyl)-2-(3-methoxy-4-(oxetan-3-yloxy)phenyl)-1H-pyrrolo[2,3-b]pyridin-5-yl)but-3-yn-1-ol (Example 6);
1-(4-(5-(4-hydroxybut-1-yn-1-yl)-3-isobutyl-1H-pyrrolo[2,3-b]pyridin-2-yl)-3,6-dihydropyridin-1(2H)-yl)ethan-1-one (Example 7);
1-(4-(5-(4-hydroxybut-1-yn-1-yl)-3-((tetrahydro-2H-pyran-4-yl)methyl)-1H-pyrrolo[2,3-b]pyridin-2-yl)-3,6-dihydropyridin-1(2H)-yl)ethan-1-one (Example 8);
4-(2-(6-methoxypyridin-3-yl)-3-((tetrahydro-2H-pyran-4-yl)methyl)-1H-pyrrolo[2,3-b]pyridin-5-yl)but-3-yn-1-ol (Example 9);

4-(3-(morpholinomethyl)-2-phenyl-1H-pyrrolo[2,3-b]pyridin-5-yl)but-3-yn-1-ol;
4-(3-(morpholinomethyl)-2-(3,4,5-trimethoxyphenyl)-1H-pyrrolo[2,3-b]pyridin-5-yl)but-3-yn-1-ol;
4-(2-(3,5-dimethoxyphenyl)-3-(morpholinomethyl)-1H-pyrrolo[2,3-b]pyridin-5-yl)but-3-yn-1-ol;
4-(2-(3-methoxyphenyl)-3-(morpholinomethyl)-1H-pyrrolo[2,3-b]pyridin-5-yl)but-3-yn-1-ol;
4-(2-(2-methoxyphenyl)-3-(morpholinomethyl)-1H-pyrrolo[2,3-b]pyridin-5-yl)but-3-yn-1-ol;
4-(2-(4-ethoxyphenyl)-3-(morpholinomethyl)-1H-pyrrolo[2,3-b]pyridin-5-yl)but-3-yn-1-ol;
4-(2-(4-isopropoxyphenyl)-3-(morpholinomethyl)-1H-pyrrolo[2,3-b]pyridin-5-yl)but-3-yn-1-ol;
4-(3-(morpholinomethyl)-2-(4-propoxyphenyl)-1H-pyrrolo[2,3-b]pyridin-5-yl)but-3-yn-1-ol;
4-(2-(6-methoxypyridin-3-yl)-3-(morpholinomethyl)-1H-pyrrolo[2,3-b]pyridin-5-yl)but-3-yn-1-ol;
4-(2-(2-methoxypyridin-5-yl)-3-(morpholinomethyl)-1H-pyrrolo[2,3-b]pyridin-5-yl)but-3-yn-1-ol;
1-(4-(5-(4-hydroxybut-1-yn-1-yl)-3-(morpholinomethyl)-1H-pyrrolo[2,3-b]pyridin-2-yl)-3,6-dihydropyridin-1(2H)-yl)ethan-1-one;

1-(5-(5-(4-hydroxybut-1-yn-1-yl)-3-(morpholinomethyl)-1H-pyrrolo[2,3-b]pyridin-2-yl)pyridin-2-yl)piperidin-4-ol;
4-(3-(morpholinomethyl)-2-(4-(oxetan-3-yloxy)phenyl)-1H-pyrrolo[2,3-b]pyridin-5-yl)but-3-yn-1-ol;
4-(3-(morpholinomethyl)-2-(3-morpholinophenyl)-1H-pyrrolo[2,3-b]pyridin-5-yl)but-3-yn-1-ol;
4-(3-(morpholinomethyl)-2-(6-morpholinopyridin-3-yl)-1H-pyrrolo[2,3-b]pyridin-5-yl)but-3-yn-1-ol;
4-(2-(6-methoxynaphthalen-2-yl)-3-(morpholinomethyl)-1H-pyrrolo[2,3-b]pyridin-5-yl)but-3-yn-1-ol;
4-(2-(3-methoxy-4-morpholinophenyl)-3-(morpholinomethyl)-1H-pyrrolo[2,3-b]pyridin-5-yl)but-3-yn-1-ol;
4-(2-(4-methoxy-3-methylphenyl)-3-(morpholinomethyl)-1H-pyrrolo[2,3-b]pyridin-5-yl)but-3-yn-1-ol;
4-(2-(3-methoxy-4-(oxetan-3-yloxy)phenyl)-3-(morpholinomethyl)-1H-pyrrolo[2,3-b]pyridin-5-yl)but-3-yn-1-ol;
1-(4-(5-(4-hydroxybut-1-yn-1-yl)-3-(morpholinomethyl)-1H-pyrrolo[2,3-b]pyridin-2-yl)-2-methoxyphenyl)piperidin-4-ol;

4-(2-(6-methoxypyridin-3-yl)-3-(piperidin-1-ylmethyl)-1H-pyrrolo[2,3-b]pyridin-5-yl)but-3-yn-1-ol;
4-(3-((3-oxa-8-azabicyclo[3.2.1]octan-8-yl)methyl)-2-(3,4-dimethoxyphenyl)-1H-pyrrolo[2,3-b]pyridin-5-yl)but-3-yn-1-ol;
4-(3-((3-oxa-8-azabicyclo[3.2.1]octan-8-yl)methyl)-2-(3-morpholinophenyl)-1H-pyrrolo[2,3-b]pyridin-5-yl)but-3-yn-1-ol;
4-(3-((3-oxa-8-azabicyclo[3.2.1]octan-8-yl)methyl)-2-(3-methoxy-4-morpholinophenyl)-1H-pyrrolo[2,3-b]pyridin-5-yl)but-3-yn-1-ol;

4-(3-((3-oxa-8-azabicyclo[3.2.1]octan-8-yl)methyl)-2-(4-morpholinophenyl)-1H-pyrrolo[2,3-b]pyridin-5-yl)but-3-yn-1-ol;
1-(4-(3-((3-oxa-8-azabicyclo[3.2.1]octan-8-yl)methyl)-S-(4-hydroxybut-1-yn-1-yl)-1H-pyrrolo[2,3-b]pyridin-2-yl)phenyl)piperidin-4-ol;
1-(4-(3-((3-oxa-8-azabicyclo[3.2.1]octan-8-yl)methyl)-(5-(4-hydroxybut-1-yn-1-yl)-1H-pyrrolo[2,3-b]pyridin-2-yl)-2-methoxyphenyl)piperidin-4-ol;
4-(3-isobutyl-2-(4-methoxyphenyl)-1H-pyrrolo[2,3-b]pyridin-5-yl)but-3-yn-1-ol;
4-(2-(4-ethoxyphenyl)-3-isobutyl-1H-pyrrolo[2,3-b]pyridin-5-yl)but-3-yn-1-ol;
4-(3-isobutyl-2-(4-morpholinophenyl)-1H-pyrrolo[2,3-b]pyridin-5-yl)but-3-yn-1-ol;
4-(3-isobutyl-2-(pyridin-4-yl)-1H-pyrrolo[2,3-b]pyridin-5-yl)but-3-yn-1-ol;
4-(3-isobutyl-2-(6-methoxypyridin-3-yl)-1H-pyrrolo[2,3-b]pyridin-5-yl)but-3-yn-1-ol;
4-(3-isobutyl-2-(2-methoxypyrimidin-5-yl)-1H-pyrrolo[2,3-b]pyridin-5-yl)but-3-yn-1-ol;
4-(3-isobutyl-2-(1,2,3,6-tetrahydropyridin-2-yl)-1H-pyrazol-4-yl)-1H-pyrrolo[2,3-b]pyridin-5-yl)but-3-yn-1-ol;
1-(4-(5-(4-hydroxybut-1-yn-1-yl)-3-isobutyl-1H-pyrrolo[2,3-b]pyridin-2-yl)-3,6-dihydropyridin-1(2H) - yl)propan-1-one;

1-(4-(5-(4-hydroxybut-1-yn-1-yl)-3-isobutyl-1H-pyrrolo[2,3-b]pyridin-2-yl)-3,6-dihydropyridin-1(2H)-yl)butan-1-one;
1-(4-(5-(4-hydroxybut-1-yn-1-yl)-3-isobutyl-1H-pyrrolo[2,3-b]pyridin-2-yl)-3,6-dihydropyridin-1(2H) - yl)2-methylpropan-1-one;
4-(2-(4-methoxyphenyl)-3-((tetrahydro-2H-pyran-4-yl)methyl)-1H-pyrrolo[2,3-b]pyridin-5-yl)but-3-yn-1-ol;
4-(2-(4-morpholinophenyl)-3-((tetrahydro-2H-pyran-4-yl)methyl)-1H-pyrrolo[2,3-b]pyridin-5-yl)but-3-yn-1-ol;
4-(2-(6-morpholinopyrimidin-3-yl)-3-((tetrahydro-2H-pyran-4-yl)methyl)-1H-pyrrolo[2,3-b]pyridin-5-yl)but-3-yn-1-ol; and
4-(2-(2-methoxypyrimidin-5-yl)-3-((tetrahydro-2H-pyran-4-yl)methyl)-1H-pyrrolo[2,3-b]pyridin-5-yl)but-3-yn-1-ol.

In Test Examples described later, it was confirmed that the following compounds exhibit potent inhibitory activities against CSF-1R:

4-(2-(4-methoxyphenyl)-3-(morpholinomethyl)-1H-pyrrolo[2,3-b]pyridin-5-yl)but-3-yn-1-ol (Example 1);
4-(3-((3-oxa-8-azabicyclo[3.2.1]octan-8-yl)methyl)-2-(4-methoxyphenyl)-1H-pyrrolo[2,3-b]pyridin-5-yl)but-3-yn-1-ol (Example 2);
4-(2-(3,4-dimethoxyphenyl)-3-(morpholinomethyl)-1H-pyrrolo[2,3-b]pyridin-5-yl)but-3-yn-1-ol (Example 3);
4-(3-(morpholinomethyl)-2-(4-morpholinophenyl)-1H-pyrrolo[2,3-b]pyridin-5-yl)but-3-yn-1-ol (Example 4);
1-(4-(5-(4-hydroxybut-1-yn-1-yl)-3-(morpholinomethyl)-1H-pyrrolo[2,3-b]pyridin-2-yl)phenyl)piperidin-4-ol (Example 5);
4-(3-((3-oxa-8-azabicyclo[3.2.1]octan-8-yl)methyl)-2-(3-methoxy-4-(oxetan-3-yloxy)phenyl)-1H-pyrrolo[2,3-b]pyridin-5-yl)but-3-yn-1-ol (Example 6);
1-(4-(5-(4-hydroxybut-1-yn-1-yl)-3-isobutyl-1H-pyrrolo[2,3-b]pyridin-2-yl)-3,6-dihydropyridin-1(2H)-yl)ethan-1-one (Example 7);
1-(4-(5-(4-hydroxybut-1-yn-1-yl)-3-((tetrahydro-2H-pyran-4-yl)methyl)-1H-pyrrolo[2,3-b]pyridin-2-yl)-3,6-dihydropyridin-1(2H)-yl)ethan-1-one (Example 8); and
4-(2-(6-methoxypyridin-3-yl)-3-((tetrahydro-2H-pyran-4-yl)methyl)-1H-pyrrolo[2,3-b]pyridin-5-yl)but-3-yn-1-ol (Example 9).

When a compound of the present invention has geometric or optical isomers, mixtures or isolates of the isomers are also encompassed in the scope of the present invention. Isomers can be separated by a conventional method.
The salt of a compound of formula (I) is not particularly limited and may be any salt that is acceptable as a pharmaceutical drug. Examples of the salt include acid addition salts of mineral acids, such as hydrochloride, hydrobromate, hydroiodate, sulfate, nitrate, and phosphate; and acid addition salts of organic acids, such as benzoate, methanesulfonate, ethanesulfonate, benzenesulfonate, p-toluenesulfonate, maleate, fumarate, tartrate, citrate, and acetate.
The solvate of a compound of formula (I) or a salt thereof is, for example, a hydrate, but is not limited thereto.
The "anticancer agent" of the present invention can be used for preventing and/or treating, for example, melanoma, sarcoma, osteosarcoma, lymphoma, leukemia, neuroblastoma, glioblastoma, neuroblastoma, giant cell tumor of tendon sheath, rhabdomyosarcoma, breast cancer, uterine cancer, oral cancer, tongue cancer, thyroid cancer, esophageal cancer, stomach cancer, colorectal cancer, colon cancer, rectal cancer, gallbladder cancer, bile duct cancer, renal cancer, renal cell carcinoma, hepatic cancer, hepatocellular carcinoma, small cell lung cancer, non-small cell lung cancer, ovarian cancer, pancreatic cancer, prostate cancer, testicular cancer, bladder cancer, or leukemia.
The compound of the present invention may be in the form of a so-called prodrug, i.e., a compound which is metabolized in vivo and converted to a compound of formula (I). Examples of the group forming a prodrug of the compound of the present invention include groups that are described in "Progress in Medicine", Life Science Medica, 1985, vol. 5, pp. 2157-2161 and groups described in "Pharmaceutical Research and Development", vol. 7, Molecular Design, pp. 163-198, published in 1990, Hirokawa Publishing Inc.
The compound of formula (I) or a salt thereof, or a solvate thereof can be manufactured by various known methods. The compound of formula (I) can be manufactured by the following general manufacturing examples 1 to 3, but the method is not particularly limited thereto. Incidentally, in the following manufacturing methods, when the defined group changes in accordance with the conditions of an implemental method or is improper for implementing the method, the compound can be manufactured through a method that is usually used in organic synthetic chemistry (for example, protection and deprotection of functional groups, see Green's Protective Groups in Organic Synthesis, Fourth Edition, John Wiley & Sons, Inc., 2006). Each process is performed by a method that is usually used in organic synthetic chemistry (for example, see Comprehensive Organic Transformations, Second Edition, John Wiley & Sons, Inc., 1999), but is not particularly limited thereto. In addition, the order of introducing substituents can be changed as needed.

[General manufacturing example 1]

When R is a morpholino group or a 3-oxa-8-azabicyclo[3.2.1]octan-8-yl group, the compound of formula (I) can be manufactured by, for example, the method described in Scheme 1:

[Scheme 1]

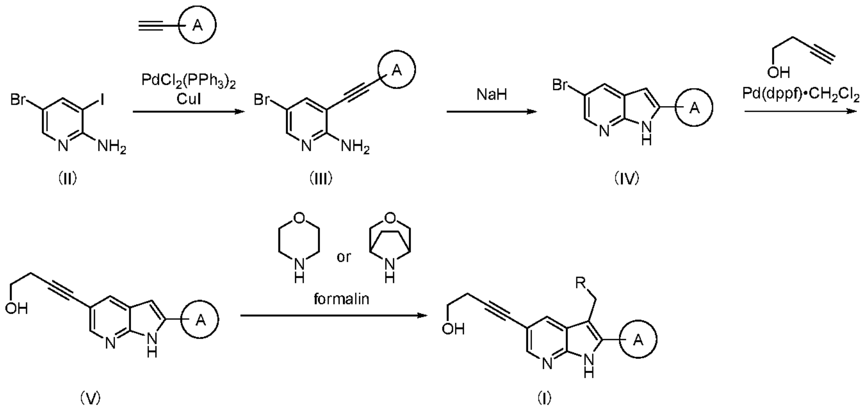


where A is synonymous with that in formula (I).

Compound (I) can be manufactured using 2-amino-5-bromo-3-iodopyridine (compound (II)) as a starting raw material. That is, an alkyne derivative having a desired substituent is reacted with compound (II) to synthesize a compound (III) by Sonogashira coupling; the compound (III) is treated with sodium hydride to synthesize compound (IV) with an azaindole ring constructed; compound (IV) is subjected to Sonogashira coupling to synthesize compound (V); and then a compound (I) can be manufactured by the Mannich reaction using formaldehyde and a secondary amine.

[General manufacturing example 2]

When R is a morpholino group or a 3-oxa-8-azabicyclo[3.2.1]octan-8-yl group, the compound of formula (I) can be manufactured by, for example, the method described in Scheme 2:

[Scheme 2]

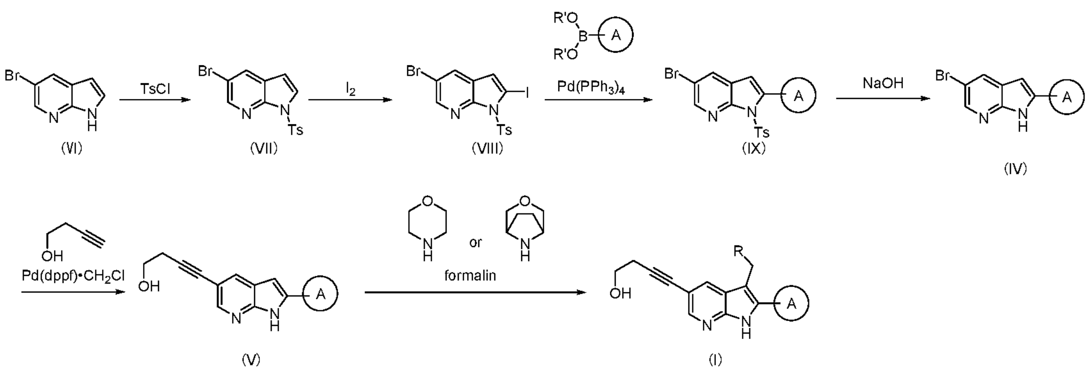


where A is synonymous with that in formula (I), and R' denotes a hydrogen atom or any functional group, such as an alkyl group.

Compound (I) can be manufactured using 5-bromo-1H-pyrrolo[2,3-b]pyridine (compound (VI)) as a starting raw material. That is, compound (VII) obtained by treatment of compound (VI) with tosyl chloride is iodinated to synthesize compound (VIII); compound (VIII) is subjected to Suzuki coupling with a boronic acid derivative having a desired substituent to synthesize compound (IX); from compound (IX), the tosyl group is removed with sodium hydroxide to synthesize compound (IV); compound (IV) is subjected to Sonogashira coupling to synthesize compound (V); and compound (I) can be manufactured by the Mannich reaction of compound (V) using formaldehyde and secondary amine.

[General manufacturing example 3]

When R is a $C_{1-3}$ alkyl group or a tetrahydro-2H-pyran-4-yl group, the compound of formula (I) can be manufactured by, for example, the method described in Scheme 3:

[Scheme 3]

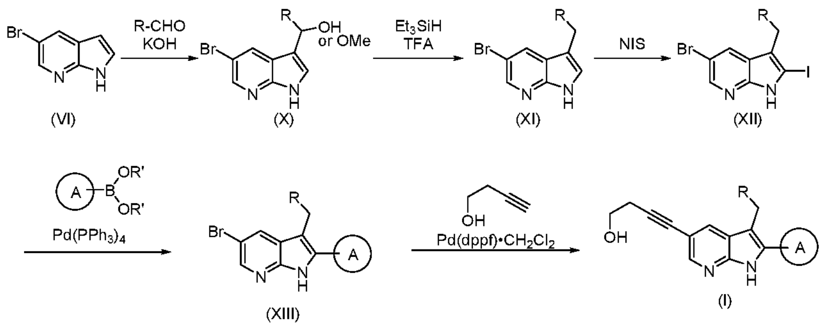


where A is synonymous with that in formula (I), and R' denotes a hydrogen atom or any functional group, such as an alkyl group.

Compound (I) can be manufactured using 5-bromo-1H-pyrrolo[2,3-b]pyridine (compound (VI)) as a starting raw material. That is, the compound (VI) is reacted with aldehyde and potassium hydroxide for alkylation by Friedel-Crafts reaction; subsequently, the generated hydroxyl group is removed by a reduction reaction using triethylsilane in trifluoroacetic acid to synthesize a compound (XI); a compound (XII) obtained by treatment of compound (XI) with N-iodosuccinimide is subjected to Suzuki coupling with a boronic acid derivative having a desired substituent to synthesize compound (XIII); and then compound (I) can be manufactured by Sonogashira coupling.

Incidentally, compound (I) having a desired substituent at a desired position can be obtained by appropriately combining the above-described methods, using a raw material corresponding to the desired substituent to be introduced (a commercial product or a compound derived from a commercially available compound by a known method or a method similar thereto), and implementing a method that is usually used in organic synthetic chemistry (for example, an alkylation reaction of an amino group, amidation of an amino group, oxidation reaction of an alkylthio group to a sulfoxide group or a sulfone group, and a reaction for converting an alkoxy group to a hydroxyl group or vice versa) by changing the order of the processes as needed.

An intermediate and a target object obtained in each reaction above can be isolated and purified as needed by subjecting to a purification method that is usually used in organic synthetic chemistry, such as filtration, extraction, washing, drying, concentration, recrystallization, and various chromatographic methods. An intermediate can also be subjected to a subsequent reaction without specifically being purified.

The compound of formula (I) or a salt thereof, or a solvate thereof can be used as a pharmaceutical composition by mixing with, for example, a carrier or an additive that is acceptable as a pharmaceutical drug. The pharmaceutical composition is prepared as an oral agent, an injection, a suppository, an ointment, an inhaler, an eye drop, a nasal drop, a patch, etc. based on a known formulation technology, and can be orally or parenterally administered to a patient.

The dosage of a compound of formula (I) or a salt thereof, or a solvate thereof can be changed in accordance with the severity of a disease, the age and body weight of a patient, the dosage form, etc. For example, it can be administered to an adult patient within a range from 1 to 1,000 mg per day, in one to several divided doses.

In addition, a radioactively labeled compound of formula (I) can be used as a molecular probe for PET.

## Examples

Hereinafter, the present invention will be more specifically described by Examples and Test Examples, but is not limited to these Examples. Each compound was identified by proton nuclear magnetic resonance spectrometry or mass spectrometry. $^1H$-NMR was measured at 400 Hz unless otherwise instructed, and exchangeable hydrogen is not clearly observed in accordance with the compound and measurement conditions in some cases. Abbreviations used in the following Examples have the following meanings.

THF: tetrahydrofuran

DMF: dimethylformamide
DMSO: dimethyl sulfoxide
NMP: N-methylpyrrolidone
s: singlet
d: doublet
t: triplet
q: quartet
dd: double doublet
m: multiplet
br: broad
J: coupling constant
Hz: Hertz
$CDCl_3$: deuterated chloroform
$CD_3OD$: deuterated methanol
DMSO-$d_6$: deuterated dimethyl sulfoxide
$^1$H-NMR: proton nuclear magnetic resonance

Example 1: Manufacture of 4-(2-(4-methoxyphenyl)-3-(morpholinomethyl)-1H-pyrrolo[2,3-b]pyridin-5-yl)but-3-yn-1-ol

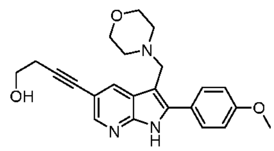

(First process)

Triethylamine (40.3 mL) was added to a THF solution (150 mL) containing 2-amino-5-bromo-3-iodopyridine (15.0 g), 1-ethynyl-4-methoxybenzene (7.30 g), bis(triphenylphosphine)palladium(II) dichloride (3.51 g), and copper(I) iodide (960 mg), and the reaction mixture was stirred in an argon atmosphere at room temperature for 17 hours. The reaction mixture was concentrated under reduced pressure and then dissolved in chloroform, and a saturated aqueous solution of ammonium chloride was then added thereto. The organic layer was separated, and the obtained organic layer was washed with a saturated saline solution and dried over anhydrous sodium sulfate, followed by concentration under reduced pressure. The resulting residue was purified by silica gel column chromatography (hexane/ethyl acetate) to give 5-bromo-3-((4-methoxyphenyl)ethynyl)pyridine-2-amine (12.0 g).

$^1$H-NMR ($CDCl_3$) $\delta$: 3.84 (3H, s), 5.04 (2H, s), 6.90 (2H, d, J = 8.8 Hz), 7.45 (2H, d, J = 8.8 Hz), 7.68 (1H, d, J = 2.0Hz), 8.06 (1H, s).

(Second process)

An NMP solution (32 mL) of 5-bromo-3-((4-methoxyphenyl)ethynyl)pyridine-2-amine (12.0 g) was dropwise added to an NMP solution (24 mL) of sodium hydride (55%, 4.84 g) at 0°C, and the reaction mixture was stirred at 80°C for 5 hours. A saturated aqueous solution of ammonium chloride was added to the reaction mixture, and the precipitated solid was collected by filtration. The obtained solid was washed with water and dried to give 5-bromo-2-(4-methoxyphenyl)-1H-pyrrolo[2,3-b]pyridine (13.1 g).

$^1$H-NMR ($CDCl_3$) $\delta$: 3.88 (3H, s), 6.60 (1H, s), 7.01 (2H, d, J = 8.4 Hz), 7.71 (2H, d, J = 9.2 Hz), 8.00 (1H, d, J = 1.6 Hz), 8.18 (1H, s).

(Third process)

1,8-Diazabicyclo[5.4.0]-7-undecene (11.1 mL) was added to a DMSO solution (60 mL) containing 5-bromo-2-(4-methoxyphenyl)-1H-pyrrolo[2,3-b]pyridine (2.25 g), 3-butyn-1-ol (2.25 mL), and [1,1'-bis(diphenylphosphino)ferrocene]palladium(II) dichloride dichloromethane adduct (606 mg), the reaction mixture was stirred in an argon atmosphere at 80°C for 16 hours. Water was added to the reaction mixture, and the precipitated solid was collected by filtration. The obtained solid was dissolved in chloroform and was then dried over anhydrous sodium sulfate, followed by concentration under reduced pressure. The resulting residue was purified by silica gel column chromatography (chloroform/methanol)

to give 4-(2-(4-methoxyphenyl)-1H-pyrrolo[2,3-b]pyridin-5-yl)but-3-yn-1-ol (2.42 g).

$^1$H-NMR ($CDCl_3$) $\delta$: 2.75 (2H, d, J = 6.4 Hz), 3.83-3.95 (5H, m), 6.62 (1H, d, J = 13.6 Hz), 7.01 (2H, d, J = 8.4 Hz), 7.65 (2H, d, J = 8.4 Hz), 7.93 (1H, s), 8.30 (1H, d, J = 2.0 Hz), 9.73 (1H, s).

(Fourth process)

An acetic acid/2-propanol solution (13 mL/13 mL) containing 4-(2-(4-methoxyphenyl)-1H-pyrrolo[2,3-b]pyridin-5-yl)but-3-yn-1-ol (1.00 g) was added to an aqueous solution of formaldehyde (37%, 0.56 mL) and an acetic acid solution (13 mL) of morpholine (0.59 mL), and the reaction mixture was stirred at room temperature for 7 hours. The reaction mixture was concentrated under reduced pressure and then dissolved in chloroform, and a saturated aqueous solution of sodium hydrogen carbonate was then added thereto. The organic layer was separated, and the obtained organic layer was washed with a saturated saline solution and dried over anhydrous sodium sulfate, followed by concentration under reduced pressure. The resulting residue was purified by silica gel column chromatography (chloroform/methanol) to give 4-(2-(4-methoxyphenyl)-3-(morpholinomethyl)-1H-pyrrolo[2,3-b]pyridin-5-yl)but-3-yn-1-ol (780 mg).

$^1$H-NMR ($CD_3OD$) $\delta$: 2.41-2.53 (4H, m), 2.66 (2H, t, J = 6.8 Hz), 3.61-3.72 (6H, m), 3.77 (2H, t, J = 6.8 Hz), 3.86 (3H, s), 7.03-7.10 (2H, m), 7.71-7.80 (2H, m), 8.13 (1H, d, J = 1.6 Hz), 8.20 (1H, s).

Example 2: Manufacture of 4-(3-((3-oxa-8-azabicyclo[3.2.1]octan-8-yl)methyl)-2-(4-methoxyphenyl)-1H-pyrrolo[2,3-b]pyridin-5-yl)but-3-yn-1-ol

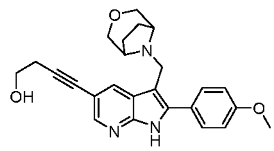

The title compound was obtained in accordance with the method of Example 1 using 3-oxa-8-azabicyclo[3.2.1]octane instead of morpholine.

$^1$H-NMR ($CD_3OD$) $\delta$ (ppm): 1.86-2.05 (4H, m), 2.76 (2H, t, J = 6.2 Hz), 3.08 (2H, s), 3.46-3.53 (2H, m), 3.64 (2H, s), 3.67-3.73 (2H, m), 3.88 (2H, t, J = 6.2 Hz), 3.91 (3H, s), 7.07 (2H, d, J = 8.8 Hz), 7.87 (2H, d, J = 8.8 Hz), 8.19 (1H, s), 8.23 (1H, d, J = 2.0 Hz), 11.2 (1H, s).

Example 3: Manufacture of 4-(2-(3,4-dimethoxyphenyl)-3-(morpholinomethyl)-1H-pyrrolo[2,3-b]pyridin-5-yl)but-3-yn-1-ol

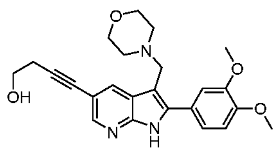

(First process)

Sodium hydride (55%, 4.32 g) was added to a DMF solution (180 mL) of 5-bromo-1H-pyrrolo[2,3-b]pyridine (15.0 g) at 0°C, followed by stirring at 0°C for 1.5 hours. Subsequently, p-toluenesulfonyl chloride (17.4 g) was added thereto, and the reaction mixture was stirred at room temperature for 1.5 hours. The reaction mixture was diluted with toluene, and water was then added thereto. The organic layer obtained by extraction with toluene was washed with a saturated saline solution. After it was dried over anhydrous sodium sulfate, concentration under reduced pressure was performed to give 5-bromo-1-tosyl-1H-pyrrolo[2,3-b]pyridine (28.8 g).

$^1$H-NMR ($CDCl_3$) $\delta$: 2.37 (3H, s), 6.52 (1H, d, J = 4.1 Hz), 7.27 (2H, d, J = 8.2 Hz), 8.72 (1H, d. J = 4.1 Hz), 7.95 (1H, d, J = 1.8 Hz), 8.03 (2H, d, J = 8.2 Hz), 8.43 (1H, d, J = 2.3 Hz) .

(Second process)

A THF/heptane/ethylbenzene solution of lithium diisopropylamide (Sigma-Aldrich Co. LLC, 2 mol/L, 18.0 mL) was added to a THF solution (225 mL) of 5-bromo-1-tosyl-1H-pyrrolo[2,3-b]pyridine (10.5 g) at -78°C, followed by stirring at -78°C for 1.5 hours. Subsequently, a THF solution (45 mL) of iodine (11.2 g) was dropwise added to the reaction mixture over 30 minutes. The reaction mixture was stirred at -78°C for 1 hour and then at 0°C for 2 hours. A saturated aqueous solution of ammonium chloride and an aqueous solution of sodium thiosulfate were added to the reaction mixture, and ethyl acetate was then added thereto. The organic layer was separated, and the obtained organic layer was washed with a saturated saline solution and dried over anhydrous sodium sulfate, followed by concentration under reduced pressure. The resulting residue was purified by silica gel column chromatography (chloroform) to give 5-bromo-2-iodo-1-tosyl-1H-pyrrolo[2,3-b]pyridine (11.8 g).

$^1$H-NMR ($CDCl_3$) δ: 2.39 (3H, s), 6.92 (1H, s), 7.29 (2H, d, J = 8.7 Hz), 7.84 (1H, d, J = 1.8 Hz), 8.07 (2H, d, J = 8.2 Hz), 8.40 (1H, d, J = 2.3 Hz).

(Third process)

Tetrakis(triphenylphosphine)palladium (2.47 g) was added to a 1,4-dioxane/water mixture solvent (250 mL/50 mL) containing 5-bromo-2-iodo-1-tosyl-1H-pyrrolo[2,3-b]pyridine (10.2 g), 3,4-dimethoxyphenylboronic acid (4.28 g), and potassium carbonate (6.91 g), and the reaction mixture was heated to reflux in an argon atmosphere for 24 hours. The reaction mixture was diluted with ethyl acetate, and water was then added thereto. The organic layer was separated, and the obtained organic layer was washed with a saturated saline solution and dried over anhydrous sodium sulfate, followed by concentration under reduced pressure. The resulting residue was purified by silica gel column chromatography (hexane/ethyl acetate) to give 5-bromo-2-(3,4-dimethoxyphenyl)-1-tosyl-1H-pyrrolo[2,3-b]pyridine (6.67 g) .

$^1$H-NMR ($CDCl_3$) δ: 2.30 (3H, s), 3.88 (3H, s), 3.93 (3H, s), 6.38 (1H, s), 6.91 (1H, d, J = 8.2 Hz), 7.02 (1H, s), 7.04 (1H, d, J = 8.2 Hz), 7.14 (2H, d, J = 7.8 Hz), 7.67 (2H, d, J = 7.3 Hz), 7.84 (1H, s), 8.44 (1H, s).

(Fourth process)

Sodium hydroxide (3.67 g) was added to a 1,4-dioxane/methanol solution (180 mL/180 mL) of 5-bromo-2-(3,4-dimethoxyphenyl)-1-tosyl-1H-pyrrolo[2,3-b]pyridine (8.94 g), and the reaction mixture was stirred at room temperature for 15 hours. The reaction mixture was concentrated under reduced pressure and then dissolved in chloroform, and a saturated aqueous solution of ammonium chloride was then added thereto. The organic layer was separated, and the obtained organic layer was washed with a saturated saline solution and dried over anhydrous sodium sulfate, followed by concentration under reduced pressure to give 5-bromo-2-(3,4-dimethoxyphenyl)-1H-pyrrolo[2,3-b]pyridine (6.32 g).

$^1$H-NMR ($CDCl_3$) δ: 3.96 (3H, s), 3.96 (3H, s), 6.60 (1H, d, J = 2.3 Hz), 7.00 (1H, d, J = 8.2 Hz), 7.23 (1H, d, J = 2.3 Hz), 7.33 (1H, dd, J = 8.2, 1.8 Hz), 8.01 (1H, d, J = 1.8 Hz), 8.25 (1H, d, J = 2.3 Hz), 11.0 (1H, s).

(Fifth process)

4-(2-(3,4-Dimethoxyphenyl)-1H-pyrrolo[2,3-b]pyridin-5-yl)but-3-yn-1-ol was obtained in accordance with the method of the third process of Example 1, using 5-bromo-2-(3,4-dimethoxyphenyl)-1H-pyrrolo[2,3-b]pyridine instead of 5-bromo-2-(4-methoxyphenyl)-1H-pyrrolo[2,3-b]pyridine.

$^1$H-NMR ($CDCl_3$) δ: 2.73 (2H, d, J = 6.2 Hz), 3.84 (2H, d, J = 6.2 Hz), 3.94 (3H,s), 3.96 (3H, s), 6.62 (1H, d, J = 1.8 Hz), 6.97 (1H, d, J = 8.7 Hz), 7.19 (1H, d, J = 1.8 Hz), 7.22-7.25 (1H, m), 7.92 (1H, d, J = 1.8 Hz), 8.30 (1H, d, J = 1.8 Hz), 9.53 (1H, s).

(Sixth process)

4-(2-(3,4-Dimethoxyphenyl)-3-(morpholinomethyl)-1H-pyrrolo[2,3-b]pyridin-5-yl)but-3-yn-1-ol was obtained in accordance with the method of the fourth process of Example 1, using 4-(2-(3,4-dimethoxyphenyl)-1H-pyrrolo[2,3-b]pyridin-5-yl)but-3-yn-1-ol instead of 4-(2-(4-methoxyphenyl)-1H-pyrrolo[2,3-b]pyridin-5-yl)but-3-yn-1-ol.

$^1$H-NMR ($CDCl_3$) δ: 2.51-2.60 (4H, m), 2.76 (2H, t, J = 6.0 Hz), 3.64 (2H, s), 3.67-3.76 (4H, m), 3.88 (2H, t, J = 6.0 Hz), 3.96 (3H, s), 3.98 (3H, s), 7.04 (1H, d, J = 8.4 Hz), 7.35 (1H, dd, J = 8.4, 1.6 Hz), 7.72 (1H, d, J = 1.6 Hz), 8.07 (1H, d, J = 2.0 Hz), 8.28 (1H, d, J = 2.0 Hz), 10.4 (1H, s).

Example 4: Manufacture of 4-(3-(morpholinomethyl)-2-(4-morpholinophenyl)-1H-pyrrolo[2,3-b]pyridin-5-yl)but-3-yn-1-ol

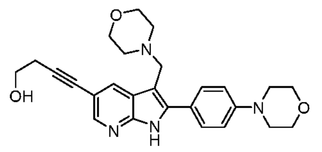

The title compound was obtained in accordance with the method of Example 3, using 4-morpholinophenylboronic acid instead of 3,4-dimethoxyphenylboronic acid.

$^1$H-NMR ($CDCl_3$) δ: 2.44-2.59 (4H, m), 2.74 (2H, t, J = 6.0 Hz), 3.17-3.38 (4H, m), 3.63 (2H, s), 3.64-3.78 (4H, m), 3.81-4.00 (6H, m), 7.03 (2H, d, J = 8.4 Hz), 7.76 (2H, d, J = 8.8 Hz), 8.08 (1H, s), 8.25 (1H, s), 10.3 (1H, s)

Example 5: Manufacture of 1-(4-(5-(4-hydroxybut-1-yn-1-yl)-3-(morpholinomethyl)-1H-pyrrolo[2,3-b]pyridin-2-yl)phenyl)piperidin-4-ol

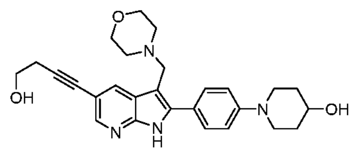

The title compound was obtained in accordance with the method of Example 3, using (4-(4-hydroxypiperidin)-1-yl)phenylboronic acid instead of 3,4-dimethoxyphenylboronic acid.

$^1$H-NMR ($CDCl_3$) δ: 1.65-1.77 (2H, m), 1.99-2.08 (2H, m), 2.48-2.55 (4H, m), 2.73 (2H, t, J = 6.4 Hz), 2.98-3.08 (2H, m), 3.64 (2H, s), 3.66-3.74 (6H, m), 3.82-3.91 (3H, m), 7.04 (2H, d, J = 8.6 Hz), 7.72 (2H, d, J = 8.6 Hz), 8.08 (1H, s), 8.24 (1H, s).

Example 6: Manufacture of 4-(3-((3-oxa-8-azabicyclo[3.2.1]octan-8-yl)methyl)-2-(3-methoxy-4-(oxetan-3-yloxy)phenyl)-1H-pyrrolo[2,3-b]pyridin-5-yl)but-3-yn-1-ol

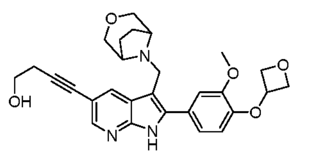

The title compound was obtained in accordance with the method of Example 3, using 3-methoxy-4-(oxetan-3-yloxy)phenylboronic acid instead of 3,4-dimethoxyphenylboronic acid and using 3-oxa-8-azabicyclo[3.2.1]octane instead of morpholine.

$^1$H-NMR ($CDCl_3$) δ: 1.91-2.12 (4H, m), 2.75 (2H, t, J = 6.4 Hz), 3.10 (2H, s), 3.49-3.55 (2H, m), 3.61-3.72 (4H, m), 3.86 (2H, t, J = 6.4 Hz), 3.98 (3H, s), 4.89-4.95 (2H, m), 5.00-5.06 (2H, m), 5.26-5.34 (1H, m), 6.61 (1H, d, J = 8.0 Hz), 7.37 (1H, dd, J = 8.0, 1.6 Hz), 7.69 (1H, d, J = 1.6 Hz), 8.12 (1H, d, J = 1.8 Hz), 8.24 (1H, d, J = 1.6 Hz), 10.9 (1H, s).

Example 7: Manufacture of 1-(4-(5-(4-hydroxybut-1-yn-1-yl)-3-isobutyl-1H-pyrrolo[2,3-b]pyridin-2-yl)-3,6-dihydropyridin-1(2H)-yl)ethan-1-one

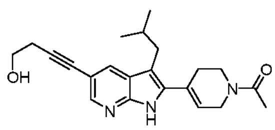

(First process)

Potassium hydroxide (71.2 g) was added to a methanol solution (1,000 mL) of 5-bromo-1H-pyrrolo[2,3-b]pyridine (50.0 g) under ice cooling, followed by stirring at room temperature for 2 hours. A methanol solution (250 mL) of isobutyraldehyde (22.0 g) was dropwise added to the reaction mixture, followed by stirring at room temperature for 17 hours. Subsequently, a methanol solution (90 mL) of isobutyraldehyde (8.10 g) was dropwise added to the reaction mixture, followed by stirring at room temperature for 4 days. The reaction mixture was concentrated under reduced pressure, and the residue was diluted with ethyl acetate, neutralized with dilute hydrochloric acid, and extracted with chloroform. The obtained organic layer was dried over sodium sulfate, and the solvent was distilled away under reduced pressure to give a crude mixture of 5-bromo-3-(1-hydroxy-2-methylpropyl)-1H-pyrrolo[2,3-b]pyridine and 5-bromo-3-(1-methoxy-2-methylpropyl)-1H-pyrrolo[2,3-b]pyridine. The resulting crude was dissolved in trifluoroacetic acid (250 mL), and triethylsilane (202 mL) was added thereto, followed by stirring at 50°C for 20 hours. The reaction mixture was concentrated under reduced pressure, and the residue was dissolved in chloroform. The organic layer was washed with a saturated aqueous solution of sodium hydrogen carbonate and a saturated saline solution and dried over anhydrous sodium sulfate, followed by concentration under reduced pressure. The resulting residue was solid washed with hexane to give 5-bromo-3-isobutyl-1H-pyrrolo[2,3-b]pyridine (38.8 g).

$^1$H-NMR ($CDCl_3$) $\delta$: 0.94 (6H, d, J = 6.4 Hz), 1.85-2.01 (1H, m), 2.55 (2H, d, J = 7.3 Hz), 7.08 (1H, s), 8.00 (1H, d, J = 1.8 Hz), 8.31 (1H, d, J = 2.3 Hz), 8.90 (1H, s) .

(Second process)

N-Iodosuccinimide (14.1 g) was added to a dichloromethane solution (250 mL) of 5-bromo-3-isobutyl-1H-pyrrolo[2,3-b]pyridine (10.6 g) at 0°C, and the mixture was stirred at 0°C for 1 hour and at 40°C for 12 hours. An aqueous solution of sodium thiosulfate was added thereto to suspend the reaction, and a saturated aqueous solution of sodium hydrogen carbonate was then added thereto for neutralization. The reaction mixture was extracted with chloroform. The obtained organic layers were combined, and the solvent was concentrated under reduced pressure. The resulting residue was washed with chloroform to give 5-bromo-2-iodo-3-isobutyl-1H-pyrrolo[2,3-b]pyridine (7.44 g).

$^1$H-NMR ($CDCl_3$) $\delta$: 0.95 (6H, d, J = 6.4 Hz), 1.87-2.08 (1H, m), 2.51 (2H, d, J = 7.3 Hz), 7.95 (1H, d, J = 2.3 Hz), 8.31 (1H, d, J = 1.8 Hz), 10.72 (1H, s).

(Third process)

Tetrakis(triphenylphosphine)palladium (3.14 g) was added to a 1,4-dioxane/water solution (240 mL/42 mL) containing 5-bromo-2-iodo-3-isobutyl-1H-pyrrolo[2,3-b]pyridine (10.0 g), 1-(tert-butoxycarbonyl)-1,2,3,6-tetrahydro-4-(4,4,5,5-tetramethyl-1,3,2-dioxaborolan-2-yl)pyridine (9.80 g), and potassium carbonate (5.80 g), and the reaction mixture was stirred in an argon atmosphere at 80°C for 12 hours. The reaction mixture was diluted with ethyl acetate, and water was then added thereto. The organic layer was separated, and the obtained organic layer was washed with a saturated saline solution and dried over anhydrous sodium sulfate, followed by concentration under reduced pressure. The resulting residue was purified by silica gel column chromatography (hexane/ethyl acetate) to give tert-butyl-4-(5-bromo-3-isobutyl-1H-pyrrolo[2,3-b]pyridin-2-yl)-3,6-dihydropyridine-1(2H)-carboxylate (8.95 g).

$^1$H-NMR ($CDCl_3$) $\delta$: 0.91 (6H, d, J = 6.9 Hz), 1.51 (9H, s), 1.82-1.97 (1H, m), 2.49-2.69 (4H, m), 3.68 (2H, t, J = 5.5 Hz), 4.05-4.22 (2H, m), 6.08 (1H, s), 7.89-8.12 (1H, m), 8.17 (1H, t, J = 1.8 Hz), 10.70 (1H, brs).

(Fourth process)

A 4N solution of hydrochloric acid in dioxane (30 mL) was added to a chloroform solution (100 mL) of tert-butyl-4-(5-bromo-3-isobutyl-1H-pyrrolo[2,3-b]pyridin-2-yl)-3,6-dihydropyridine-1(2H)-carboxylate (8.95 g), and the reaction mixture was stirred at room temperature for 4 hours. The reaction mixture was concentrated under reduced pressure. The residue was diluted with chloroform and then neutralized with a saturated aqueous solution of sodium hydrogen carbonate, followed by extraction with chloroform. The obtained organic layer was washed with a saturated saline solution, dried over anhydrous sodium sulfate, and then concentrated under reduced pressure to give crude 5-bromo-3-isobutyl-2-(1,2,3,6-tetrahydropyridin-4-yl)-1H-pyrrolo[2,3-b]pyridine (6.42 g).

(Fifth process)

Pyridine (3.62 mL) and acetyl chloride (1.91 mL) were added to a dichloromethane solution (80 mL) of the crude 5-bromo-3-isobutyl-2-(1,2,3,6-tetrahydropyridin-4-yl)-1H-pyrrolo[2,3-b]pyridine (3.0 g) at 0°C, and the reaction mixture was stirred at room temperature for 21 hours. The reaction mixture was concentrated under reduced pressure,

and methanol (10 mL) and an aqueous solution of sodium hydroxide were added to the resulting residue. The reaction mixture was stirred at room temperature for 5 hours. An aqueous solution of 1N hydrochloric acid was added to the reaction mixture for neutralization, followed by extraction with chloroform. The obtained organic layer was washed with a saturated saline solution and dried over anhydrous sodium sulfate, followed by concentration under reduced pressure. The resulting residue was purified by silica gel column chromatography (silica gel, ethyl acetate/methanol) to give 1-(4-(5-bromo-3-isobutyl-1H-pyrrolo[2,3-b]pyridin-2-yl)-3,6-dihydropyridin-1(2H)-yl)ethan-1-one (2.63 g).

$^1$H-NMR ($CDCl_3$) δ: 0.93 (6H, d, J = 5.9 Hz), 1.84-2.04 (1H, m), 2.11-2.28 (3H, m), 2.58-2.77 (4H, m), 3.69-3.95 (2H, m), 4.20-4.44 (2H, m), 6.03-6.20 (1H, m), 7.95 (1H, s), 8.22 (1H, s), 9.72 (1H, brs)

(Sixth process)

3-Butyn-1-ol (2.08 mL), [1,1'-bis(diphenylphosphino)ferrocene]palladium(II) dichloride dichloromethane adduct (846 mg), and piperidine (6.8 mL) were added to an acetonitrile/DMSO solution (60 mL/12 mL) of 1-(4-(5-bromo-3-isobutyl-1H-pyrrolo[2,3-b]pyridin-2-yl)-3,6-dihydropyridin-1(2H)-yl)ethan-1-one (2.62 g).

The reaction mixture was stirred in an argon atmosphere at 80°C for 3 hours. The reaction mixture was diluted with ethyl acetate, and water was then added thereto. After extraction with ethyl acetate, the obtained organic layer was washed with a saturated saline solution and dried over anhydrous sodium sulfate, followed by concentration under reduced pressure. The resulting residue was purified by silica gel column chromatography (ethyl acetate/methanol) to give 1-(4-(5-(4-hydroxybut-1-yn-1-yl)-3-isobutyl-1H-pyrrolo[2,3-b]pyridin-2-yl)-3,6-dihydropyridin-1(2H)-yl)ethan-1-one (660 mg).

$^1$H-NMR ($CDCl_3$) δ: 0.91 (6H, d, J = 6.4 Hz), 1.92 (1H, m), 2.15-2.20 (3H, m), 2.56-2.66 (4H, m), 2.72 (2H, t, J = 5.6 Hz), 3.67-3.72 (1H, m), 3.81-3.88 (3H, m), 4.17-4.22 (1H, m), 4.28-4.33 (1H, m), 5.99-6.11 (1H, m), 7.86-7.90 (1H, brs), 8.23-8.28 (1H, brs), 9.35-9.67 (1H, m).

Example 8: Manufacture of 1-(4-(5-(4-hydroxybut-1-yn-1-yl)-3-((tetrahydro-2H-pyran-4-yl)methyl)-1H-pyrrolo[2,3-b]pyridin-2-yl)-3,6-dihydropyridin-1(2H)-yl)ethan-1-one

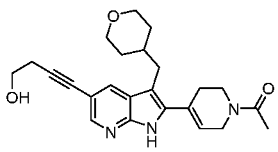

The title compound was obtained in accordance with the method of Example 7, using tetrahydro-2H-pyran-4-carbaldehyde instead of isobutyraldehyde.

$^1$H-NMR ($CDCl_3$) δ: 1.28-1.42 (2H, m), 1.49-1.58 (2H, m), 1.71-1.86 (1H, m), 2.18-2.21 (3H, m), 2.66-2.75 (4H, m), 3.30 (2H, m), 3.72 (1H, t, J = 6.0 Hz), 3.83-3.96 (4H, m), 4.19-4.37 (2H, m), 6.01-6.14 (1H, m), 7.87-7.90 (1H, m), 8.22-8.25 (1H, m), 10.6-10.85 (1H, m).

Example 9: Manufacture of 4-(2-(6-methoxypyridin-3-yl)-3-((tetrahydro-2H-pyran-4-yl)methyl)-1H-pyrrolo[2,3-b]pyridin-5-yl)but-3-yn-1-ol

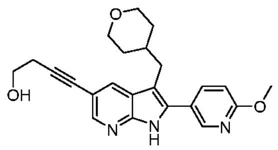

The title compound was obtained in accordance with the method of Example 7, using tetrahydro-2H-pyran-4-carbaldehyde instead of isobutyraldehyde and using 6-methoxy-3-(4,4,5,5-tetramethyl-1,3,2-dioxaborolan-2-yl)pyridine instead of 1-(tert-butoxycarbonyl)-1,2,3,6-tetrahydro-4-(4,4,5,5-tetramethyl-1,3,2-dioxaborolan-2-yl)pyridine.

$^1$H-NMR ($CD_3OD$) δ: 1.19-1.31 (2H, m), 1.48-1.57 (2H, m), 1.77-1.93 (1H, m), 2.66 (2H, t, J = 6.6 Hz), 2.79 (2H, d, J = 7.2 Hz), 3.24-3.32 (2H, m), 3.77 (2H, t, J = 6.6 Hz), 3.80-3.90 (2H, m), 3.98 (3H, s), 6.94 (1H, dd, J = 8.8 Hz), 7.91 (1H, dd, J = 8.6, 2.6 Hz), 8.03 (1H, d, J = 1.6 Hz), 8.22 (1H, s), 8.38 (1H, d, J = 2.0Hz).

The compounds shown in Table 1 to Table 6 were obtained in accordance with the methods described in Examples 1 to 9 above.

[Table 1]

| Structure | Data |
|---|---|
| 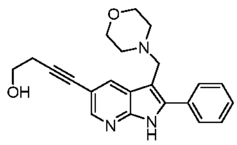 | $^1$H-NMR (DMSO-$d_6$) δ: 2.38-2.45 (4H, m), 2.60 (2H, t, J = 6.8 Hz), 3.53-3.67 (8H, m), 4.92 (1H, t, J = 5.6 Hz), 7.39-7.45 (1H, m), 7.49-7.55 (2H, m), 7.86-7.92 (2H, m), 8.12 (1H, d, J = 2.2 Hz), 8.24 (1H, d, J = 2.2 Hz), 12.1 (1H, s). |
| 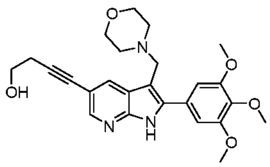 | $^1$H-NMR (CDCl$_3$) δ: 2.51-2.58 (4H, m), 2.73 (2H, t, J = 6.3 Hz), 3.63 (2H, s), 3.64-3.75 (4H, m), 3.86 (2H, t, J = 6.3 Hz), 3.91 (6H, s), 3.94 (3H, s), 7.30 (2H, s), 8.05 (1H, s), 8.21 (1H, s), 12.2 (1H, brs). |
| 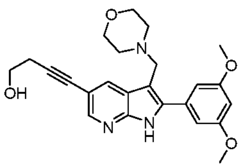 | $^1$H-NMR (CDCl$_3$) δ: 2.51-2.58 (4H, m), 2.75 (2H, t, J = 6.3 Hz), 3.63-3.75 (6H, m), 3.82-3.90 (8H, m), 6.56 (1H, s), 7.18-7.19 (2H, m), 8.09 (1H, s), 8.32 (1H, s), 12.0 (1H, brs). |
| 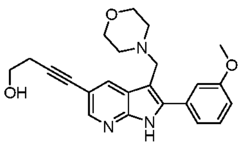 | $^1$H-NMR (CDCl$_3$) δ: 2.47-2.58 (4H, m), 2.75 (2H, t, J = 5.9 Hz), 3.62-3.75 (6H, m), 3.83-3.92 (5H, m), 6.97-7.04 (1H, m), 7.40-7.48 (2H, m), 7.63 (1H, s), 8.12 (1H, s), 8.27 (1H, s), 11.8 (1H, brs). |
| 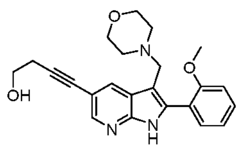 | $^1$H-NMR (CDCl$_3$) δ: 2.42-2.54 (4H, m), 2.74 (2H, t, J = 6.3 Hz), 3.61 (2H, s), 3.65-3.72 (4H, m), 3.83-3.89 (5H, m), 7.03 (1H, d, J = 8.6 Hz), 7.08 (1H, t, J = 7.3 Hz), 7.40 (1H, t, J = 7.7 Hz), 7.94 (1H, d, J = 7.7 Hz), 8.13 (1H, s), 8.27 (1H, s), 9.99 (1H, brs). |
| 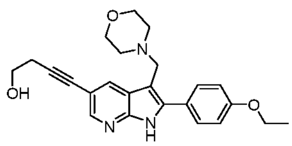 | $^1$H-NMR (CDCl$_3$) δ: 1.48 (3H, t, J = 6.8 Hz), 2.47-2.59 (4H, m), 2.76 (2H, t, J = 6.2 Hz), 3.61-3.79 (6H, m), 3.88 (2H, t, J = 6.2 Hz), 4.13 (2H, q, J = 7.2 Hz), 7.05 (2H, d, J = 8.8 Hz), 7.77 (2H, d, J = 8.8 Hz), 8.11 (1H, s), 8.28 (1H, d, J = 2.0 Hz), 10.2 (1H, s). |
| 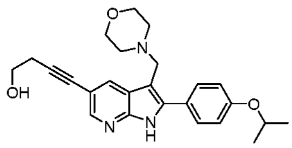 | $^1$H-NMR (CDCl$_3$) δ: 1.41 (6H, d, J = 6.0 Hz), 2.46-2.58 (4H, m), 2.76 (2H, t, J = 6.4 Hz), 3.65 (2H, s), 3.68-3.80 (4H, m), 3.88 (2H, t, J = 6.4 Hz), 4.61-4.72 (1H, m), 7.04 (2H, d, J = 8.4 Hz), 7.77 (2H, d, J = 8.4 Hz), 8.11 (1H, d, J = 1.8 Hz), 8.26 (1H, d, J = 1.8 Hz), 10.6 (1H, s). |
| 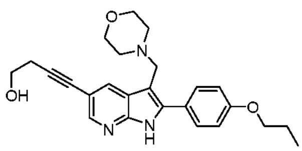 | $^1$H-NMR (CDCl$_3$) δ: 1.07 (3H, t, J = 7.2 Hz), 1.80-1.93 (2H, m), 2.43-2.57 (4H, m), 2.75 (2H, t, J = 6.3 Hz), 3.62 (2H, s), 3.63-3.75 (4H, m), 3.86 (2H, t, J = 6.3 Hz), 4.00 (2H, t, J = 6.8 Hz), 7.01-7.08 (2H, m), 7.72-7.79 (2H, m), 8.09 (1H, s), 8.25 (1H, s), 10.4 (1H, brs). |

[Table 2]

| Structure | Data |
|---|---|
| 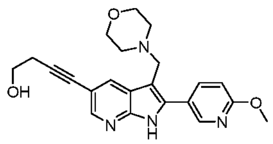 | $^1$H-NMR ($CDCl_3$) δ: 2.42-2.63 (4H, m), 2.76 (2H, t, J = 6.4 Hz), 3.60-3.68 (2H, m), 3.69-3.79 (4H, m), 3.82-3.95 (2H, m), 4.03 (3H, s), 6.91 (1H, d. J = 8.0 Hz), 8.04-8.19 (2H, m), 8.31 (1H, s), 8.62 (1H, s), 10.1 (1H, s). |
| 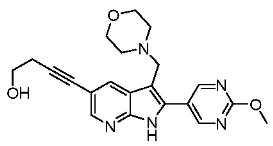 | $^1$H-NMR ($CDCl_3$) δ: 2.49-2.57 (4H, m), 2.77 (2H, t, J = 5.6 Hz), 3.60 (2H, s), 3.67-3.75 (4H, m), 3.86-3.92 (2H, m), 4.14 (3H, s), 8.12 (1H, s), 8.32 (1H, s), 9.09 (2H, s). |
| 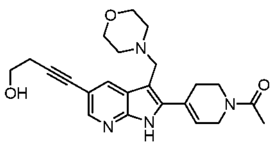 | $^1$H-NMR ($CDCl_3$) δ: 2.15-2.28 (3H, m), 2.39-2.60 (4H, m), 2.65-2.79 (4H, m), 3.53-3.80 (8H, m), 3.86-3.96 (3H, m), 4.22-4.44 (2H, m), 6.42 (1H, s), 8.07-8.09 (1H, m), 8.25-8.29 (1H, m), 10.7-10.9 (1H, m). |
| 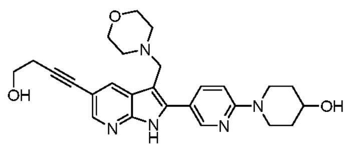 | $^1$H-NMR ($CDCl_3$) δ: 1.48-1.60 (2H, m), 1.89-1.98 (2H, m), 2.42-2.55 (4H, m), 2.64 (2H, t, J = 6.9 Hz), 3.15-3.25 (2H, m), 3.61 (2H, s), 3.63-3.71 (4H, m), 3.75 (2H, t, J = 6.9 Hz), 3.81-3.89 (1H, m), 4.06-4.15 (2H, m), 6.87 (1H, d, J = 9.2 Hz), 7.68 (1H, s), 7.94 (1H, d, J = 9.2 Hz), 8.04 (1H, s), 8.17 (1H, s), 8.56 (1H, s). |
| 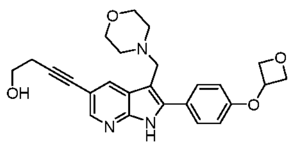 | $^1$H-NMR ($CDCl_3$) δ: 2.47-2.53 (4H, m), 2.73 (2H, t, J = 6.2 Hz), 3.63 (2H, s), 3.68-3.75 (4H, m), 3.84 (2H, t, J = 6.2 Hz), 4.80-4.85 (2H, m), 5.01-5.07 (2H, m), 5.25-5.33 (1H, m), 6.83 (2H, d, J = 8.0 Hz), 7.77 (2H, d, J = 8.0 Hz), 8.11 (1H, s), 8.25 (1H, s). |
| 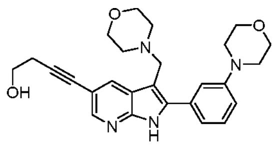 | $^1$H-NMR ($CDCl_3$) δ: 2.50-2.59 (4H, m), 2.77 (2H, t, J = 6.2 Hz), 3.25-3.30 (4H, m), 3.67 (2H, s), 3.68-3.74 (4H, m), 3.85-3.94 (6H, m), 7.00 (1H, dd, J = 8.0, 2.2 Hz), 7.31 (1H, d, J = 7.2 Hz), 7.44 (1H, t, J = 8.0 Hz), 7.62-7.66 (1H, m), 8.12 (1H, d, J = 2.0 Hz), 8.31 (1H, d, J = 2.0 Hz), 11.0 (1H, s). |
| 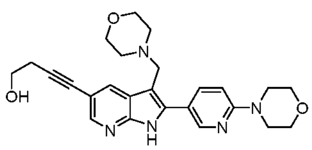 | $^1$H-NMR ($CDCl_3$) δ: 2.43-2.58 (4H, m), 2.74 (2H, t, J = 6.0 Hz), 3.55-3.64 (6H, m), 3.65-3.74 (4H, m), 3.81-3.90 (6H, m), 6.76 (1H, d, J = 8.7 Hz), 7.99 (1H, d, J = 8.0 Hz), 8.08 (1H, s), 8.27 (1H, s), 8.63 (1H, s), 10.3 (1H, brs). |

[Table 3]

| Structure | Data |
|---|---|
| 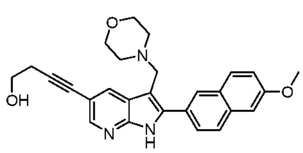 | $^1$H-NMR ($CDCl_3$) δ: 1.82-1.95 (4H, m), 2.41 (2H, d, J = 10.8 Hz), 2.57-2.64 (2H, m), 2.74 (2H, t, J = 6.4 Hz), 3.57 (2H, s), 3.85 (2H, t, J = 6.4 Hz), 3.89 (3H, s), 4.30 (2H, s), 7.03 (2H, d, J = 8.8 Hz), 7.85 (2H, d, J = 8.8 Hz), 8.05 (1H, s), 8.24 (1H, s), 10.8 (1H, s). |
| 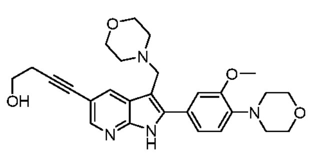 | $^1$H-NMR ($CDCl_3$) δ: 1.87-1.96 (1H, m), 2.46-2.61 (4H, m), 2.75 (2H, t, J = 6.0 Hz), 3.10-3.20 (4H, m), 3.58-3.74 (6H, m), 3.81-3.98 (9H, m), 7.03 (1H, d, J = 6.0 Hz), 7.29-7.31 (1H, m), 7.64 (1H, s), 8.05 (1H, s), 8.31 (1H, s), 9.58 (1H, brs). |

(continued)

| Structure | Data |
|---|---|
| 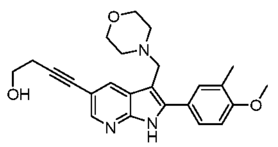 | 1H-NMR ($CDCl_3$) δ: 2.46-2.58 (4H, m), 2.75 (2H, t, J = 6.3 Hz), 3.63 (2H, s), 3.65-3.75 (4H, m), 3.86 (2H, t, J = 6.3 Hz), 3.92 (3H, s), 6.98 (1H, d, J = 8.7 Hz), 7.69-7.75 (2H, m), 8.09 (1H, s), 8.23 (1H, s), 11.6 (1H, brs). |
| 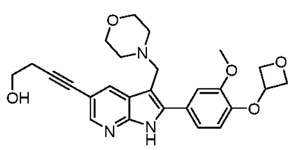 | 1H-NMR ($CDCl_3$) δ: 2.51-2.59 (4H, m), 2.74 (2H, t, J = 6.4 Hz), 3.63 (2H, s), 3.68-3.75 (4H, m), 3.85 (2H, t, J = 6.4 Hz), 6.97 (3H, s), 4.88-4.94 (2H, m), 4.99-5.05 (2H, m), 5.25-5.33 (1H, m), 6.60 (1H, d, J = 8.0 Hz), 7.28-7.33 (1H, m), 7.73 (1H, d, J = 1.6 Hz), 8.07 (1H, d, J = 2.0 Hz), 8.27 (1H, d, J = 2.0 Hz). |
| 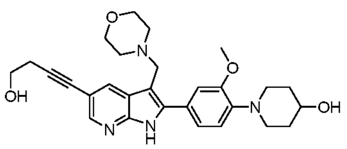 | 1H-NMR (DMSO-$d_6$) δ: 1.48-1.61 (2H, m), 1.80-1.89 (2H, m), 2.41-2.49 (4H, m), 2.59 (2H, t, J = 6.8 Hz), 2.68-2.77 (2H, m), 3.24-3.32 (2H, m), 3.53-3.65 (9H, m), 3.88 (3H, s), 4.66 (1H, d, J = 4.8 Hz), 4.92 (1H, t, J = 5.6 Hz), 7.00 (1H, d, J = 8.0 Hz), 7.40 (1H, dd, J = 8.0, 2.0 Hz), 7.73 (1H, d, J = 2.0 Hz), 8.05 (1H, d, J = 1.6 Hz), 8.20 (1H, d, J = 2.0 Hz), 12.1 (1H, s). |
| 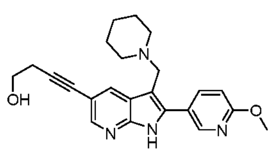 | 1H-NMR ($CDCl_3$) δ: 1.38-1.50 (2H, m), 1.50-1.62 (4H, m), 2.32-2.56 (4H, m), 2.76 (2H, t, J = 6.4 Hz), 3.51-3.64 (2H, m), 3.87 (2H, t, J = 6.4 Hz), 4.04 (3H, s), 6.91 (1H, d. J = 8.8 Hz), 8.09-8.20 (2H, m), 8.31 (1H, d, J = 2.0 Hz), 8.65 (1H, s), 11.0 (1H, s). |

[Table 4]

| Structure | Data |
|---|---|
| 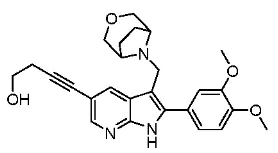 | 1H-NMR ($CDCl_3$) δ: 1.92-1.97 (2H, m), 2.06-2.11 (2H, m), 2.77 (2H, t, J = 6.0 Hz), 3.11 (2H, s), 3.52 (2H, d, J = 10.0 Hz), 3.62 (2H, s), 3.69 (2H, d, J = 10.0 Hz), 3.87 (2H, dd, J = 6.4, 12.4 Hz), 3.97 (3H, s), 3.98 (3H, s), 7.02 (1H, d, J = 8.4 Hz), 7.38 (1H, dd, J = 2.0, 8.4 Hz), 7.63 (1H, s), 8.13 (1H, s), 8.28 (1H, d, J = 2.0 Hz), 10.1 (1H, s). |
| 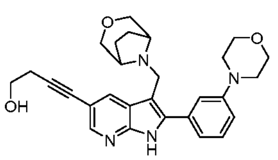 | 1H-NMR ($CDCl_3$) δ: 1.89-2.08 (4H, m), 2.75 (2H, t, J = 6.0 Hz), 3.09 (2H, s), 3.22-3.29 (4H, m), 3.45-3.53 (2H, m), 3.62-3.71 (4H, m), 3.83-3.92 (6H, m), 6.96-7.02 (1H, m), 7.35-7.39 (1H, m), 7.40-7.46 (1H, m), 7.57 (1H, s), 8.18 (1H, s), 8.27 (1H, s), 11.4 (1H, s). |
| 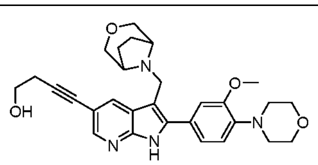 | 1H-NMR ($CDCl_3$) δ: 2.18-2.57 (4H, m), 2.73 (2H, t, J = 6.3 Hz), 3.04-3.22 (6H, m), 3.45-3.53 (2H, m), 3.60-3.71 (4H, m), 3.85 (2H, t, J = 6.3 Hz), 3.89-3.98 (7H, m), 7.04 (1H, d, J = 8.15 Hz), 7.40-7.42 (1H, m), 7.61 (1H, s), 8.13 (1H, s), 8.20 (1H, s), 11.2 (1H, brs). |
| 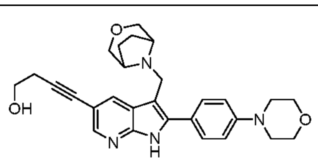 | 1H-NMR ($CDCl_3$) δ: 1.85-2.05 (4H, m), 2.75 (2H, t, J = 6.4 Hz), 3.08 (2H, s), 3.25-3.31 (4H, m), 3.46-3.53 (2H, m), 3.62-3.72 (4H, m), 3.87 (2H, t, J = 6.4 Hz), 3.89-3.93 (4H, m), 7.04 (2H, d, J = 8.8 Hz), 7.81 (2H, d, J = 8.8 Hz), 8.17 (1H, s), 8.25 (1H, d, J = 2.0 Hz), 10.5(1H, s). |
| 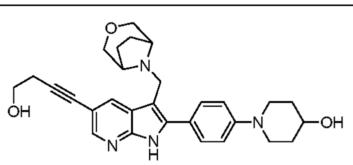 | 1H-NMR (DMSO-$d_6$) δ: 1.38-1.48 (2H, m), 1.67-1.83 (4H, m), 1.88-1.99 (2H, m), 2.54 (2H, t, J = 6.4 Hz), 2.86-2.95 (2H, m), 2.98 (2H, s), 3.34-3.46 (4H, m), 3.51 (2H, s), 3.54-3.68 (5H, m), 4.66 (1H, d, J = 4.0 Hz), 4.88 (1H, t, J = 5.6 Hz), 7.01 (2H, d, J = 7.8 Hz), 7.80 (2H, d, J = 7.8 Hz), 7.98 (1H, s), 8.13 (1H, s), 11.9 (1H, s). |

(continued)

| Structure | Data |
|---|---|
| 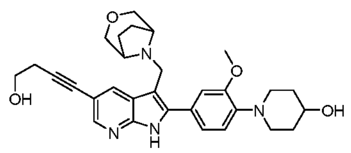 | $^1$H-NMR ($CDCl_3$) δ: 1.74-1.85 (2H, m), 1.88-1.96 (2H, m), 2.01-2.11 (4H, m), 2.71-2.78 (2H, m), 2.80-2.89 (2H, m), 3.10 (2H, s), 3.37-3.54 (5H, m), 3.62-3.71 (4H, m), 3.78-3.88 (3H, m), 3.96 (3H, s), 7.03-7.08 (1H, m), 7.36-7.41 (1H, m), 7.54 (1H, s), 8.13 (1H, s), 8.23-8.27 (1H, m). |

[Table 5]

| Structure | Data |
|---|---|
| 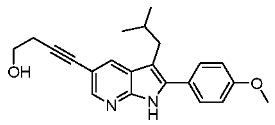 | $^1$H-NMR ($CD_3OD$) δ: 0.87 (6H, d, J = 6.6 Hz), 1.88-2.02 (1H, m), 2.59-2.79 (4H, m), 3.76 (2H, t, J = 6.8 Hz), 3.86 (3H, s), 7.05 (2H, d, J = 8.7 Hz), 7.55 (2H, d, J = 8.7 Hz), 7.96 (2H, s). |
| 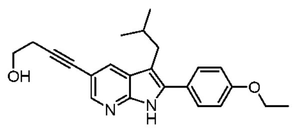 | $^1$H-NMR ($CDCl_3$) δ: 0.80 (6H, d, J = 6.6 Hz), 1.47 (3H, t, J = 6.9 Hz), 1.85-2.09 (2H, m), 2.62-2.81 (4H, m), 3.86 (2H, t, J = 6.2 Hz), 4.11 (2H, q, J = 6.9 Hz), 7.01 (2H, d, J = 8.7 Hz), 7.53 (2H, d, J = 8.7 Hz), 7.93 (1H, d, J = 1.5 Hz), 8.25 (1H, d, J = 1.5 Hz), 9.83 (1H, brs). |
| 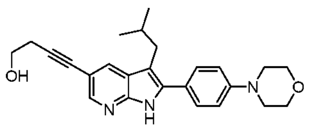 | $^1$H-NMR ($CD_3OD$) δ: 0.88 (6H, d, J = 6.4 Hz), 1.91-2.03 (1H, m), 2.66 (2H, t, J = 6.6 Hz), 2.73 (2H, d, J = 7.2 Hz), 3.20-3.26 (4H, m), 3.76 (2H, t, J = 6.6 Hz), 3.83-3.91 (4H, m), 7.08 (2H, d, J = 8.8 Hz), 7.53 (2H, d, J = 8.4 Hz), 7.93 (1H, d, J = 1.6 Hz), 8.13 (1H, s). |
| 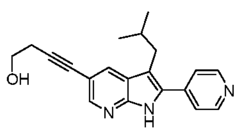 | $^1$H-NMR ($CDCl_3$) δ: 0.91 (6H, d, J = 6.3 Hz), 1.96-2.07 (1H, m), 2.70-2.81 (4H, m), 3.86 (2H, t, J = 5.9 Hz), 7.51 (2H, d, J = 6.3 Hz), 8.00 (1H, d, J = 1.8 Hz), 8.34 (1H, d, J = 1.8 Hz), 8.73 (2H, d, J = 6.3 Hz), 9.59 (1H, brs). |
| 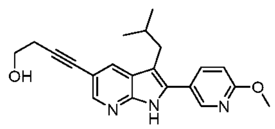 | $^1$H-NMR ($CDCl_3$) δ: 0.90 (6H, d, J = 6.4 Hz), 1.88-1.97 (1H, m), 1.98-2.09 (1H, m), 2.69 (2H, d, J = 7.6 Hz), 2.75 (2H, t, J = 6.2 Hz), 3.81-3.92 (2H, m), 4.03 (3H, s), 6.91 (1H, d, J = 8.8 Hz), 7.83 (1H, dd, J = 8.8, 2.4 Hz), 7.97 (1H, d, J = 1.6 Hz), 8.25 (1H, d, J = 2.0 Hz), 8.46 (1H, d, J = 2.4 Hz), 10.4 (1H, brs). |
| 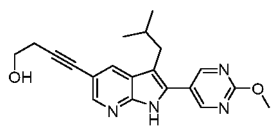 | $^1$H-NMR ($CD_3OD$) δ: 0.90 (6H, d, J = 6.8 Hz), 1.89-2.00 (1H, m), 2.66 (2H, t, J = 6.6 Hz), 2.71 (2H, d, J = 7.6 Hz), 3.76 (2H, t, J = 6.6 Hz), 4.09 (3H, s), 8.03 (1H, d, J = 2.0 Hz), 8.25 (1H, d, J = 1.6 Hz), 8.81 (2H, s). |
| 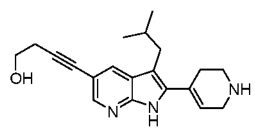 | $^1$H-NMR ($CDCl_3$) δ: 0.93 (6H, d, J = 6.8 Hz), 1.88-2.00 (1H, m), 2.44-2.52 (2H, m), 2.63 (2H, d, J = 7.3 Hz), 2.74 (2H, t, J = 5.9 Hz), 3.13 (2H, t, J = 5.9 Hz), 3.58-3.62 (2H, m), 3.85 (2H, t, J = 5.9 Hz), 6.08-6.13 (1H, m), 7.87 (1H, d, J = 1.8 Hz), 8.28 (1H, d, J = 1.8 Hz), 8.64 (1H, brs). |
| 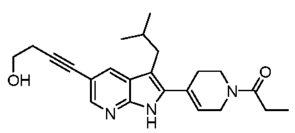 | $^1$H-NMR ($CDCl_3$) δ: 0.90 (6H, d, J = 6.8 Hz), 1.15-1.27 (3H, m), 1.84-1.98 (1H, m), 2.35-2.52 (2H, m), 2.56-2.68 (4H, m), 2.72 (2H, t, J = 5.9), 3.69-3.84 (4H, m), 4.16-4.23 (0.9H, m), 4.29-4.37 (1.1H, m), 6.02-6.07 (0.45H, m), 6.10-6.16 (0.55H, m), 7.88 (1H, s), 8.23 (1H, s), 10.8 (0.45H, brs), 11.0 (0.55H, brs). |
| 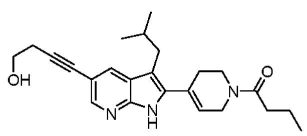 | $^1$H-NMR ($CDCl_3$) δ: 0.91 (6H, d, J = 6.8 Hz), 0.95-1.05 (3H, m), 1.67-1.78 (2H, m), 1.87-1.98 (1H, m), 2.31-2.45 (2H, m), 2.56-2.68 (4H, m), 2.72 (2H, t, J = 5.9 Hz), 3.67-3.92 (4H, m), 4.15-4.23 (0.9H, m), 4.29-4.37 (1.1H, m), 5.99-6.07 (0.45H, m), 6.08-6.15 (0.55H, m), 7.89 (1H, s), 8.25 (1H, s), 10.0 (0.45H, brs), 10.2 (0.55H, brs). |

[Table 6]

| Structure | Data |
|---|---|
| 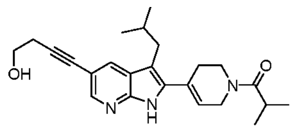 | $^1$H-NMR ($CDCl_3$) δ: 0.91 (6H, d, J = 6.3 Hz), 1.13-1.21 (6H, m), 1.85-1.99 (1H, m), 2.53-2.68 (4H, m), 2.74 (2H, t, J = 5.9 Hz), 2.78-2.94 (1H, m), 3.72-3.91 (4H, m), 4.21-4.36 (2H, m), 6.00-6.07 (0.45H, m), 6.09-6.15 (0.55H, m), 7.89 (1H, s), 8.25 (1H, s), 10.0 (0.45H, brs), 10.5 (0.55H, brs). |
| 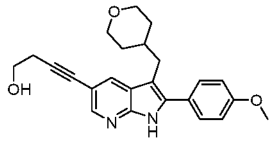 | $^1$H-NMR ($CDCl_3$) δ: 1.22-1.38 (2H, m), 1.50-1.59 (2H, m), 1.80-1.89 (1H, m), 1.89-1.99 (1H, m), 2.71-2.84 (4H, m), 3.22-3.34 (2H, m), 3.82-3.96 (7H, m), 7.06 (2H, dd, J = 6.8, 2.0 Hz), 7.55 (2H, dd, J = 6.8, 2.0 Hz), 7.93 (1H, d, J = 1.6 Hz), 8.24 (1H, d, J = 2.0 Hz), 10.3 (1H, brs). |
| 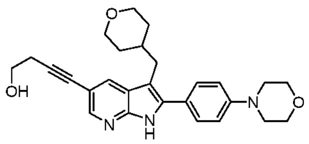 | $^1$H-NMR ($CDCl_3$) δ: 1.23-1.39 (2H, m), 1.50-1.58 (2H, m), 1.81-1.92 (1H, m), 1.92-1.99 (1H, m), 2.73-2.81 (4H, m), 3.23-3.34 (6H, m), 3.83-3.94 (8H, m), 7.02 (2H, d, J = 8.8 Hz), 7.51 (2H, d, J = 8.8 Hz), 7.91 (1H, d, J = 1.6 Hz), 8.27 (1H, d, J = 2.0 Hz), 9.61 (1H, brs). |
| 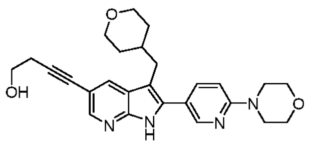 | $^1$H-NMR ($CD_3OD$) δ: 1.21-1.32 (2H, m), 1.49-1.58 (2H, m), 1.80-1.93 (1H, m), 2.66 (2H, t, J = 6.6 Hz), 2.79 (2H, d, J = 7.2 Hz), 3.23-3.30 (2H, m), 3.52-3.61 (4H, m), 3.76 (2H, t, J = 6.8 Hz), 3.78-3.89 (6H, m), 6.95 (1H, d, J = 8.8 Hz), 7.82 (1H, dd, J = 9.2, 2.4 Hz), 8.00 (1H, d, J = 2.0 Hz), 8.19 (1H, d, J = 2.0 Hz), 8.38 (1H, d, J = 2.0 Hz). |
| 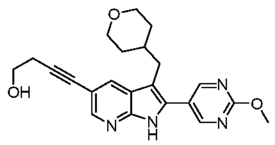 | $^1$H-NMR ($CD_3OD$) δ: 1.20-1.34 (2H, m), 1.49-1.59 (2H, m), 1.81-1.95 (1H, m), 2.66 (2H, t, J = 6.6 Hz), 2.80 (2H, d, J = 7.2 Hz), 3.31-3.38 (2H, m), 3.77 (2H, t, J = 6.6 Hz), 3.81-3.91 (2H, m), 4.11 (3H, s), 8.12 (1H, d, J = 2.0 Hz), 8.27 (1H, s), 8.82 (2H, s). |

Test Example 1: Evaluation of CSF-1R inhibitory activity

The CSF-1R inhibitory activity of an inventive compound was evaluated by a TR-FRET method using phosphorylation of a substrate peptide of CSF-1R as the index.

(Preparation and addition of test compound)

The inventive compound was dissolved in dimethyl sulfoxide sterilized by filtration to prepare a 10 μmol/L solution. This solution was dispensed using an acoustic noncontact type nanoliter dispenser (Echo 550, LABCYTE Inc.) such that the final concentrations were within a range from 0.25 to 180 nmol/L.

(Measurement of CSF-1R inhibitory activity by TR-FRET method)

The assay was performed using an HTRF™ KinEASETM-TK kit (Cisbio Bioassays, 62TK0PEC) by the procedure in accordance with the manual (62TK0PEC rev04(2009)). In the assay, a 384-well plate (Greiner 784076 Black 384 well, Small volume, Greiner Bio-One International GmbH) was used. The enzyme was Fms, active (14-551, Eurofins DiscoverX Products, LLC), which was prepared such that the final concentration was 0.05 μg/mL. TR-FRET measurement (fluorescence of 620 nm and 665 nm) was performed using an HTRF-dedicated microplate reader (K-101, Kyoritsu Radio Service Co., Ltd.). The proportion of the fluorescence quantity at each wavelength as the index of phosphorylation [((quantity of 665 nm fluorescence)/(quantity of 620 nm fluorescence)) × 10,000] was calculated, and the $IC_{50}$ value was determined by statistical treatment of the proportion of the fluorescence quantity at each compound concentration.

(Evaluation results)

The inhibitory activities in the evaluation of representative compounds of the present invention by the TR-FRET method are shown in Table 7 (in the evaluation by the TR-FRET method, the inhibitory activity was shown by *** when

the $IC_{50}$ value was less than 10 nM, was shown by ** when the $IC_{50}$ value was 10 nM or more and less than 30 nM, and was shown by * when the $IC_{50}$ value was 30 nM or more and less than 100 nM). The inventive compounds showed strong inhibitory activities against CSF-1R in the evaluation by the TR-FRET method.

[Table 7] Table showing the CSF-1R inhibitory activity

| Test compound (Example No.) | CSF-1R inhibitory activity |
|---|---|
| 1 | * |
| 2 | ** |
| 3 | ** |
| 4 | *** |
| 5 | *** |
| 6 | ** |
| 7 | *** |
| 8 | *** |
| 9 | *** |

Test Example 2: Antitumor effect in human non-small cell lung cancer cell line NCI-H460 subcutaneously transplanted nude mouse model

The antitumor effect of the inventive compound was examined using a human non-small cell lung cancer cell line NCI-H460 subcutaneously transplanted nude mouse model.

(Culture of cells)

A cell culture medium was prepared by adding 5.6 mL of Penicillin-Streptomycin (Sigma-Aldrich Co. LLC), 56 mL of FBS (MP Biomedicals), 5.6 mL of 1 mol/L HEPES (Sigma-Aldrich Co. LLC), 5.6 mL of 100 mmol/L Sodium Pyruvate (Life Technologies), and 1.4 g of D-(+)-Glucose (Wako Pure Chemical Industries, Ltd.) to a RPMI1640 culture medium (Sigma-Aldrich Co. LLC). NCI-H460 cells (American Type Culture Collection) were cultured using the prepared culture medium at 37°C in a 5% $CO_2$ incubator.

(Production of cancer-bearing model)

The cultured NCI-H460 cells were suspended in PBS. The obtained cell suspension was mixed with Matrigel (Corning Inc.) of one-third of the amount of the cell suspension to prepare a cell suspension of $5\times10^6$ cells/mL, and 0.2 mL of this cell suspension was subcutaneously injected in the left inguinal region of each BALB/c-nu/nu mouse (female, 7-week old, CHARLES RIVER LABORATORIES JAPAN, INC.). Two days after cell transplantation, grouping was performed by block allocation using the body weights of the cancer-bearing mice as the index.

(Preparation of administration solution)

An aqueous solution of 20% (2-hydroxypropyl)-β-cyclodextrin (HPβCD) (FUJIFILM Wako Pure Chemical Corporation) was added to the compound of Example 1 to prepare a 30 mg/mL sample solution for administration, which was used as the administration solution for Example 1 (300 mg/kg) group. The administration solution for Control group was the aqueous solution of 20% HPβCD. These administration solutions were prepared at time of use.

(Administration of test substance)

From the day of grouping, the administration solutions were administered by gavage to mice transplanted with cancer cells (15 mice in each group) for the total of 22 days, once a day on the day of the grouping and the last day and twice a day on other days. The administration volume was 10 mL/kg in each group. The administration volume was calculated from the last body weight of each.

(Evaluation of antitumor effect)

The tumor volume of each mouse was calculated by the following equation, and the antitumor effect was evaluated using the tumor volume as the index.

$$\text{Tumor volume (mm}^3\text{)} = \text{(major axis)} \times \text{(minor axis)} \times \text{(minor axis)}/2$$

$$\text{Tumor volume change (mm}^3\text{)} = \text{(tumor volume on each measurement day)} - \text{(tumor volume on the day after the start date of administration)}$$

$$\text{Tumor growth inhibition rate (TGI) (\%)} = \{1 - [\text{(average value of tumor volume changes in each administration group)}/\text{(average value of tumor volume changes in Control group)}]\} \times 100$$

As shown in Figure 1, the compound of Example 1, which is an inventive compound, exhibited a significant tumor growth inhibition, and the TGI% on the last administration day was 61% in 300 mg/kg. This demonstrates that the compound according to the present invention exhibited an antitumor effect and demonstrated that the compound is useful for cancer therapy.

Industrial Applicability

The azaindole derivative provided by the present invention has a CSF-1R inhibitory activity and is useful as an anticancer agent, and therefore has industrial applicability.

## Claims

**1.** A compound of formula (I)

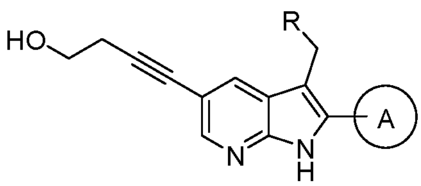

(I)

wherein

A represents a $C_{6-10}$ aryl ring, an aromatic heterocycle, or an unsaturated heterocycle, and A optionally has one substituent or more same or different substituents; and
R represents a $C_{1-3}$ alkyl group or a saturated heterocyclic group,

or a salt thereof or a solvate thereof.

**2.** The compound or the salt thereof, or the solvate thereof according to Claim 1, wherein A is a benzene ring, a naphthalene ring, a pyridine ring, a pyrimidine ring, a 1,2-dihydropyridine ring, a 1,2,3,4-tetrahydropyridine ring, or a 1,2,3,6-tetrahydropyridine ring; and the substituent of A is one to three selected from the group consisting of a $C_{1-3}$ alkyl group, a $C_{1-3}$ alkyloxy group, a $C_{1-3}$ alkylcarbonyl group, a hydroxypiperidinyl group, an oxetanyloxy group, and a morpholinyl group.

**3.** The compound or the salt thereof, or the solvate thereof according to Claim 1 or 2, wherein R is a $C_{1-3}$ alkyl group, a tetrahydropyran ring, a morpholine ring, or a 3-oxa-8-azabicyclo[3.2.1]octane ring.

**4.** The compound or the salt thereof, or the solvate thereof according to any one of Claims 1 to 3, wherein

the compound of formula (I) is
4-(2-(4-methoxyphenyl)-3-(morpholinomethyl)-1H-pyrrolo[2,3-b]pyridin-5-yl)but-3-yn-1-ol,
4-(3-((3-oxa-8-azabicyclo[3.2.1]octan-8-yl)methyl)-2-(4-methoxyphenyl)-1H-pyrrolo[2,3-b]pyridin-5-yl)but-3-yn-1-ol,
4-(2-(3,4-dimethoxyphenyl)-3-(morpholinomethyl)-1H-pyrrolo[2,3-b]pyridin-5-yl)but-3-yn-1-ol,
4-(3-(morpholinomethyl)-2-(4-morpholinophenyl)-1H-pyrrolo[2,3-b]pyridin-5-yl)but-3-yn-1-ol,
1-(4-(5-(4-hydroxybut-1-yn-1-yl)-3-(morpholinomethyl)-1H-pyrrolo[2,3-b]pyridin-2-yl)phenyl)piperidin-4-ol,
4-(3-((3-oxa-8-azabicyclo[3.2.1]octan-8-yl)methyl)-2-(3-methoxy-4-(oxetan-3-yloxy)phenyl)-1H-pyrrolo[2,3-b]pyridin-5-yl)but-3-yn-1-ol,
1-(4-(5-(4-hydroxybut-1-yn-1-yl)-3-isobutyl-1H-pyrrolo[2,3-b]pyridin-2-yl)-3,6-dihydropyridin-1(2H)-yl)ethan-1-one,
1-(4-(5-(4-hydroxybut-1-yn-1-yl)-3-((tetrahydro-2H-pyran-4-yl)methyl)-1H-pyrrolo[2,3-b]pyridin-2-yl)-3,6-dihydropyridin-1(2H)-yl)ethan-1-one, or
4-(2-(6-methoxypyridin-3-yl)-3-((tetrahydro-2H-pyran-4-yl)methyl)-1H-pyrrolo[2,3-b]pyridin-5-yl)but-3-yn-1-ol.

**5.** A pharmaceutical composition comprising the compound or the salt thereof, or the solvate according to any one of Claims 1 to 3.

**6.** A CSF-1R inhibitor comprising the compound or the salt thereof, or the solvate thereof according to any one of Claims 1 to 3 as an active component.

**7.** An anticancer agent comprising the compound or the salt thereof, or the solvate thereof according to any one of Claims 1 to 3 as an active component.

**8.** The anticancer agent according to Claim 7, which is an agent for preventing and/or treating melanoma, sarcoma, osteosarcoma, lymphoma, leukemia, neuroblastoma, glioblastoma, neuroblastoma, giant cell tumor of tendon sheath, rhabdomyosarcoma, breast cancer, uterine cancer, oral cancer, tongue cancer, thyroid cancer, esophageal cancer, stomach cancer, colorectal cancer, colon cancer, rectal cancer, gallbladder cancer, bile duct cancer, renal cancer, renal cell carcinoma, hepatic cancer, hepatocellular carcinoma, small cell lung cancer, non-small cell lung cancer, ovarian cancer, pancreatic cancer, prostate cancer, testicular cancer, bladder cancer, or leukemia.

**9.** Use of the compound or the salt thereof, or the solvate thereof according to any one of Claims 1 to 3 for manufacturing a CSF-1R inhibitor.

**10.** Use of the compound or the salt thereof, or the solvate thereof according to any one of Claims 1 to 3 for manufacturing an anticancer agent.

**11.** The compound or the salt thereof, or the solvate thereof according to any one of Claims 1 to 3 for inhibiting CSF-1R.

**12.** The compound or the salt thereof, or the solvate thereof according to any one of Claims 1 to 3 for preventing or treating cancer.

**13.** A method for inhibiting CSF-1R, comprising administering the compound or the salt thereof, or the solvate thereof according to any one of Claims 1 to 3 to a patient in need thereof.

**14.** A method for treating cancer, comprising administering the compound or the salt thereof, or the solvate thereof according to any one of Claims 1 to 3 to a patient in need thereof.

[Figure 1]

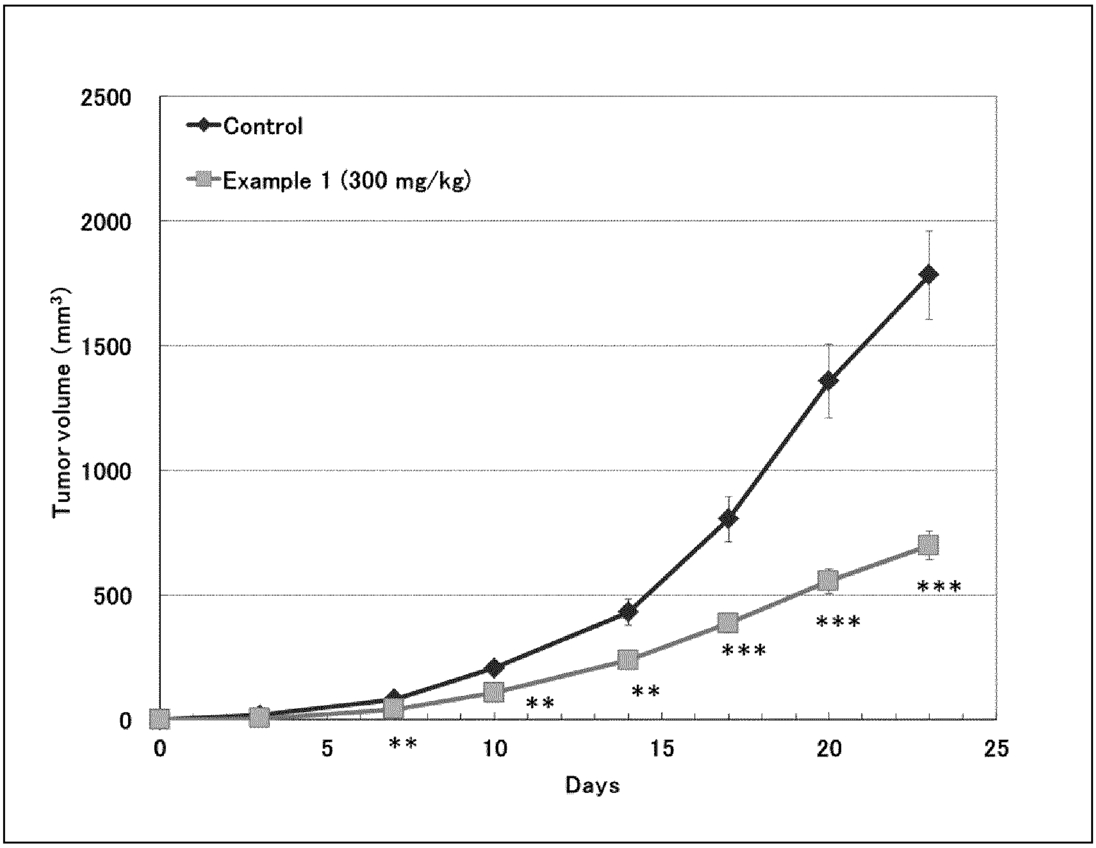
2500
2000
1500
1000
500
0
Tumor volume (mm3)
Control
Example 1 (300 mg/kg)
**
**
**
***
***
***
0
5
10
15
20
25
Days

## INTERNATIONAL SEARCH REPORT

International application No.
PCT/JP2020/014475

A. CLASSIFICATION OF SUBJECT MATTER

Int. Cl. A61P35/00(2006.01)i, A61P35/02(2006.01)i, A61P43/00(2006.01)i, C07D471/04(2006.01)i, C07D519/00(2006.01)i, A61K31/437(2006.01)i, A61K31/4427(2006.01)i, A61K31/506(2006.01)i, A61K31/5377(2006.01)i, A61K31/5386(2006.01)i
FI: C07D471/04 102, A61K31/437, A61K31/4427, A61K31/506, A61K31/5377, A61P35/00, A61P35/02, A61P43/00 111, A61K31/5386, C07D519/00 301

According to International Patent Classification (IPC) or to both national classification and IPC

B. FIELDS SEARCHED

Minimum documentation searched (classification system followed by classification symbols)
Int. Cl. A61P35/00, A61P35/02, A61P43/00, C07D471/04, C07D519/00, A61K31/437, A61K31/4427, A61K31/506, A61K31/5377, A61K31/5386

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| A | JP 2018-524381 A (GENZYME CORP.) 30 August 2018, claims, paragraph [0002], examples 1-56, 1-58 | 1-14 |
| A | BARBERIS et al., Discovery of N-substituted 7-azaindoles as Pan-PIM kinases inhibitors -Lead optimization- Part III, Bioorganic & Medicinal Chemistry Letters, 10 December 2018, 29, pp. 491-495, ISSN: 0960-894X, in particular, abstract, scheme 2, table 1 | 1-14 |
| A | JP 2017-531678 A (JANSSEN PHARMACEUTICA NV) 26 October 2017, claims, paragraph [0001] | 1-14 |

☒ Further documents are listed in the continuation of Box C.　　☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 29.05.2020 | 09.06.2020 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

## INTERNATIONAL SEARCH REPORT

International application No. PCT/JP2020/014475

C (Continuation). DOCUMENTS CONSIDERED TO BE RELEVANT

| Category* | Citation of document, with indication, where appropriate, of the relevant passages | Relevant to claim No. |
|---|---|---|
| A | LEBOHO et al., Double Sonogashira reactions on dihalogenated aminopyridines for the assembly of an array of 7-azaindoles bearing triazole and quinoxaline substituents at C-5: Inhibitory bioactivity against Giardia duodenalis trophozoites, Bioorganic & Medicinal Chemistry, 20 May 2015, vol. 23, pp. 4943-4951, ISSN: 0968-0896, in particular, scheme 1 | 1-14 |

Form PCT/ISA/210 (continuation of second sheet) (January 2015)

**INTERNATIONAL SEARCH REPORT**
Information on patent family members

International application No.
PCT/JP2020/014475

| Patent Documents referred to in the Report | Publication Date | Patent Family | Publication Date |
|---|---|---|---|
| JP 2018-524381 A | 30.08.2018 | US 2019/0016707 A1<br>claims, paragraph [0002], examples 1-56, 1-58<br>WO 2017/015267 A1<br>EP 3325471 A1<br>CN 107922396 A<br>KR 10-2018-0030199 A | |
| JP 2017-531678 A | 26.10.2017 | US 2017/0334915 A1<br>claims, paragraph [0001]<br>US 2019/0169198 A1<br>WO 2016/062790 A1<br>EP 3209663 A1<br>KR 10-2017-0068489 A<br>CN 107074882 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011107553 A
- WO 2009075344 A
- WO 2005062795 A
- WO 2006009755 A


### Non-patent literature cited in the description


- *Critical Reviews in Clinical Laboratory Sciences,* 2012, vol. 49, 49-61
- *Current Opinion in Immunology,* 2006, vol. 18, 39-48
- *New England Journal of Medicine,* 2015, vol. 373, 428-437
- *Cell,* 2010, vol. 141, 39-51
- *Frontiers in Immunology,* 2014, vol. 5 (489), 1-15
- *Current Opinion in Pharmacology,* 2015, vol. 23, 45-51
- *Proc. Natl. Acad. Sci. USA,* 2006, vol. 103, 690-695
- *Proc. Natl. Acad. Sci. USA,* 1990, vol. 87, 1377-1380
- *Nature Medicine,* 2010, vol. 16, 580-585
- Progress in Medicine. *Life Science Medica,* 1985, vol. 5, 2157-2161
- Pharmaceutical Research and Development. Molecular Design. Hirokawa Publishing Inc, 1990, vol. 7, 163-198
- Green's Protective Groups in Organic Synthesis. John Wiley & Sons, Inc, 2006
- Comprehensive Organic Transformations. John Wiley & Sons, Inc, 1999
- 62TK0PEC rev04. 2009